# EUROPEAN PATENT SPECIFICATION

(11) **EP 0 423 802 B1**
(45) Date of publication and mention of the grant of the patent: **09.08.1995**
(21) Application number: 90120052.7
(22) Date of filing: 19.10.1990
(51) Int. Cl.: C07D 233/22, A61K 31/415, C07D 405/06, C07D 405/12, C07D 239/06, A61K 31/505, A61K 31/36

(54) **Phenoxy-heterocyclic compounds**
Phenoxyheterozyklische Verbindungen
Composés phénoxyhétérocycliques

(30) Priority: 20.10.1989 US 424429
(43) Date of publication of application: 24.04.1991
(73) Proprietor: MERRELL DOW PHARMACEUTICALS INC., Cincinnati Ohio 45215-6300 (US)
(72) Inventor: Freedman, Jules, Cincinnati, Ohio 45242 (US); Dudley, Mark W., Hamilton, Ohio 45013 (US)
(74) Representative: VOSSIUS & PARTNER

(56) References cited:
- EP-A- 0 011 596
- DE-C- 614 596
- FR-A- 2 388 496
- GB-A- 2 045 240
- US-A- 2 893 993
- US-A- 3 897 431

## Description

The present invention is directed to a new class of phenoxy-heterocyclic compounds possessing therapeutic properties. Another aspect of the invention is directed to the said compounds for use in a method for the treatment of depression, anxiety, and hypertension. A further aspect of the invention is directed to pharmaceutical compositions containing these phenoxy-heterocyclic compounds.

In accordance with the present invention, a new class of phenoxy-heterocyclic compounds have been discovered which can be described by the following formula I: 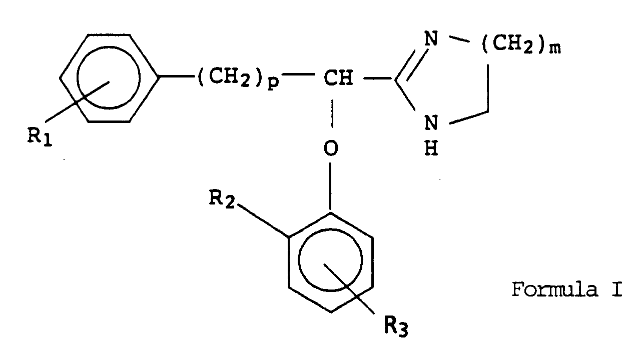
in which R₁ is represented by substituent(s) selected from the group consisting of hydrogen, halogen, C₁₋₄ alkyl, C₁₋₄ alkoxy, OH, CF₃, CN, methylenedioxy, ethylenedioxy, C₃₋₅ methylene, 2,3-benzo, 3,4-benzo, alkylthio, alkylsulfonyl, alkylsulfinyl, C₃₋₆ cycloalkyl, C₅₋₈ cycloalkoxy, and C₅₋₈ cycloalkylthio; R₂ is represented by halogen, C₁₋₄ alkyl, C₁₋₄ alkoxy, OH, CF₃, CN, alkylthio, alkylsulfinyl, C₃₋₆ cycloalkyl, C₅₋₈ cycloalkoxy or C₅₋₈ cycloalkylthio; R₃ is represented by hydrogen, halogen, C₁₋₄ alkyl, C₁₋₄ alkoxy, OH, CF₃, CN, methylenedioxy, ethylenedioxy, C₃₋₅ methylene, 2,3 benzo, 3,4-benzo, 4,5-benzo, 5,6-benzo, alkylthio, alkylsulfonyl, alkylsulfinyl, C₃₋₆ cycloalkyl, C₅₋₈ cycloalkoxy, or C₅₋₈ cycloalkylthio; m is represented by the integer 1 or 2; p is represented by an integer from 0-4; and the pharmaceutically acceptable addition salts thereof.

The compounds of Formula I exhibit multiple pharmacological properties. They are serotonin 5HT_{1A} agonists. The compounds also have an affinity for the α-2 receptor. Due to these pharmacological properties, the compounds are useful in the treatment of depression, anxiety, and hypertension.

As used in this application:
a) the term "halogen" refers to a fluorine, chlorine, or bromine atom;
b) the terms "lower alkyl group and C₁₋₄ alkyl" refer to a branched or straight chained alkyl group containing from 1-4 carbon atoms, such as methyl, ethyl, n-propyl, isopropyl, n-butyl, isobutyl, etc.;
c) the terms "lower alkoxy group and C₁₋₄ alkoxy" refer to a straight or branched alkoxy group containing from 1-4 carbon atoms, such as methoxy, ethoxy, n-propoxy, isopropoxy, n-butoxy, isobutoxy, etc.;
d) the term "methylenedioxy" refers to the following substituent: -O-CH₂-O-;
e) the term "ethylenedioxy" refers to the following substituent: -O-(CH₂)₂-O-;
f) the term "C₃₋₅ methylene" refers to the following substituent: -(CH₂)ₙ-, in which n is represented by an integer from 3 to 5;
g) the term "alkylthio" refers to the following substituent: -S-Alk, in which Alk is represented by a C₁₋₄ alkyl;
h) the term "alkylsulfonyl" refers to the following substituent: -SO₂-Alk, in which Alk is represented by a C₁₋₄ alkyl, and;
i) the term "alkylsulfinyl" refers to the following substituent: -SO-Alk, in which Alk is represented by a C₁₋₄ alkyl;
j) the term "pharmaceutically acceptable addition salt" refers to either a basic addition salt or an acid addition salt;
k) the term " C₃₋₆ cycloalkyl" refers to a cycloalkyl substituent containing from 3-6 carbon atoms such as cyclopropyl, cyclobutyl, cyclopentyl, or cyclohexyl;
l) the term "C₅₋₈ cycloalkoxy" refers to a cycloalkoxy substituent containing from 5-8 carbon atoms such as cyclopentyloxy, cyclohexyloxy, cycloheptyloxy, or cyclooctyloxy;
m) the term "C₅₋₈ cycloalkylthio" refers to a cycloalkylthio substituent containing from 5-8 carbon atoms such as cyclopentylthio, cyclohexylthio, cycloheptylthio, or cyclooctylthio.

The expression "pharmaceutically acceptable acid addition salts" is intended to apply to any non-toxic organic or inorganic acid addition salt of the base compounds represented by Formula I or any of its intermediates. Illustrative inorganic acids which form suitable salts include hydrochloric, hydrobromic, sulphuric, and phosphoric acid and acid metal salts such as sodium monohydrogen orthophosphate, and potassium hydrogen sulfate. Illustrative organic acids which form suitable salts include the mono-, di-, and tricarboxylic acids. Illustrative of such acids are for example, acetic, glycolic, lactic, pyruvic, malonic, succinic, glutaric, fumaric, malic, tartaric, citric, ascorbic, maleic, hydroxymaleic, benzoic, hydroxy-benzoic, phenylacetic, cinnamic, salicyclic, 2-phenoxy-benzoic, p-toluenesulfonic acid, and sulfonic acids such as methane sulfonic acid and 2-hydroxyethane sulfonic acid. Such salts can exist in either a hydrated or substantially anhydrous form. In general, the acid addition salts of these compounds are soluble in water and various hydrophilic organic solvents, and which in comparison to their free base forms, generally demonstrate higher melting points.

The expression "pharmaceutically acceptable basic addition salts" is intended to apply to any non-toxic organic or inorganic basic addition salts of the compounds represented by Formula I or any of its intermediates. Illustrative bases which form suitable salts include alkali metal hydroxides such as sodium or potassium hydroxide.

All of the compounds of Formula I contain at least one asymmetric center and therefore exist as enantiomers. Any reference in this application to one of the compounds represented by Formula I is meant to encompass either a specific enantiomer or a mixture of enantiomers. The specific enantiomers can be separated and recovered by techniques known in the art such as chromatography on chiral stationary phases or resolution via chiral salt formation and subsequent separation by selective crystallization.

As is apparent to those skilled in the art, the heterocyclic moiety contains a tautomeric center. Thus the double bond contained within this heterocyclic moiety will move freely between the two nitrogen atoms. Any reference in this application to one of the compounds of Formula I should be construed as encompassing either of these tautomers.

The compounds of Formula I contain two phenyl rings. One of these rings may be optionally substituted as indicated by the definition for R₁. When R₁ is other than a hydrogen atom, there can be up to 5 monovalent substituents occurring on the indicated phenyl ring. These substituents can be the same or different and can be located at any of the ortho, meta, or para positions. If R₁ is to be represented by a divalent substituent, such as a methylenedioxy moiety, an ethylenedioxy moiety, a benzo moiety or a C₃₋₅ methylene moiety, then there can be one of these substituents bonded to the indicated phenyl ring. This phenyl ring may optionally be substituted with one other non-hydrogen monovalent substituent. The divalent substituent can be bonded to either positions 2 and 3 or to positions 3 and 4 of the phenyl ring. Thus the phenyl ring and the divalent substituent will form a bicyclic ring system. These bicyclic ring systems are illustrated below in order to further exemplify the present invention. 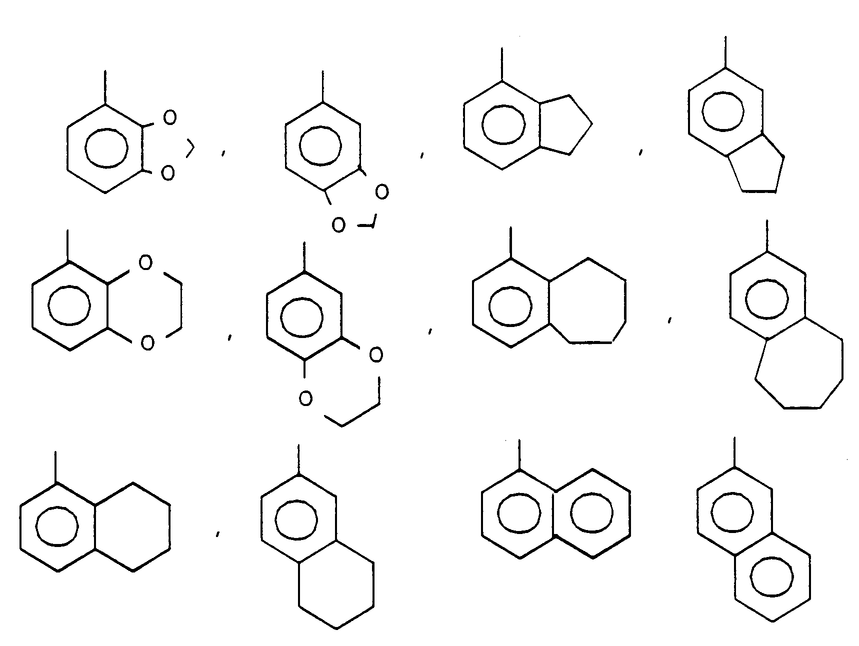
The other phenyl ring must be substituted at the 2-position as is indicated by the definition for R₂. This phenyl ring may optionally be further substituted as is indicated by the definition for R₃. When R₃ is other than a hydrogen atom, there can be up to 4 monovalent substituents bonded to this phenyl ring. These substituents may be located at any of positions 3, 4, 5, or 6. These substituents may be the same or different. R₃ may also be represented by a divalent substituent as noted above. This divalent substituent will form ring systems similar to those depicted above except that the divalent substituent may be bonded to positions 3 and 4, positions 4 and 5, or positions and 6. Only one divalent substituent may be bonded to this phenyl ring.

Examination of Formula I shows that the heterocyclic moiety can be represented by either a 5-membered ring or a 6-membered ring. Thus, these compounds can contain either a imidazoline radical or a tetrahydro-pyrimidine radical.

Illustrative compounds encompassed by Formula I include:
a) 2-[1-(2-ethoxyphenoxy)-2-phenylethyl]imidazoline
b) 2-[α-(2-methoxyphenoxy)benzyl]imidazoline
c) 2-[α-(2-ethoxyphenoxy)benzyl]imidazoline
d) 2-[1-(2-methoxyphenoxy)-2-phenylethyl]imidazoline
e) 2-[1-(2,3-dimethoxyphenoxy)-2-phenylethyl]imidazoline
f) 2-[1-(2-fluoro-4-methoxyphenoxy)-3-phenyl]propylimidazoline
g) 2-[3-(4-chlorophenyl)-1-(2-methoxyphenoxy)propyl]-1,2,3,4-tetrahydropyrimidine
h) 2-[1-(2-methoxyphenoxy)-3-phenylpropyl]imidazoline
i) 2-(1-(2-ethoxyphenoxy)-3-phenylpropyl]imidazoline
The preferred compounds of the instant invention are those in which p is represented by 1. It is also preferred for R₂ to be represented by an alkoxy residue and more preferably an ethoxy residue. It is also preferred for R₃ to be represented by hydrogen and m to be represented by 1.

The compounds of Formula I can be synthesized using techniques that are known in the art. One method for synthesizing these compounds is disclosed below in Reaction Scheme I. 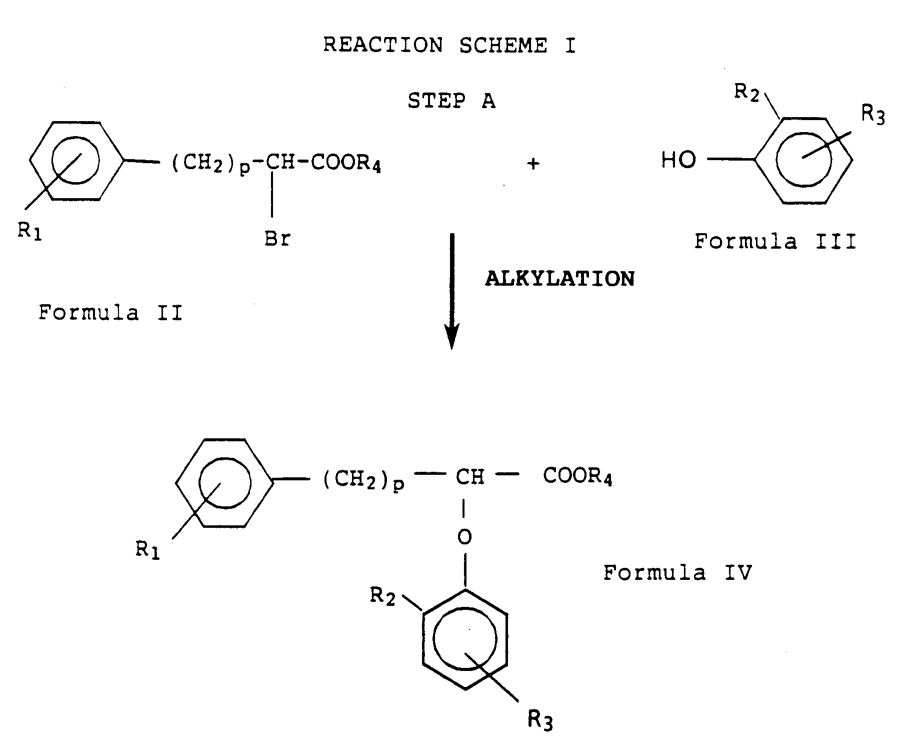
The first step in the reaction sequence is to conduct an alkylation reaction between a bromo ester as described by Formula II and a phenol as described by Formula III. In Formula II, p and R₁ are as in Formula I and R₄ is represented by a C₁₋₄ alkyl. In Formula III, R₂ and R₃ are as in Formula I.

The appropriate starting materials are a bromo ester in which p and R₁ have the same definitions as that desired in the final product and a phenol in which R₂ and R₃ have the same definitions as that appearing in the final product. The particular C₁₋₄ alkyl which is present at the R₄ position is immaterial since this substituent will not be retained in the final product.

The alkylation reaction can be conducted utilizing techniques well known in the art. Approximately equimolar amounts of the bromo ester of Formula II and the phenol of Formula III are contacted in an organic solvent such as acetone or benzene. The reactants are typically contacted in the presence of a base such as K₂CO₃. This base is typically present in a molar excess. The reactants are then heated to reflux and the reaction is allowed to proceed for a period of time ranging from about 10 to 96 hours.

The resulting oxy ester intermediate of Formula IV can be recovered from the reaction medium and purified using techniques known in the art. The oxy ester intermediate is typically recovered by concentration as is known in the art. This oxy ester intermediate can then be purified by either distillation or by recrystallization from a solvent such as pentane or hexane using techniques known in the art.

As depicted below in Step B of Reaction Scheme I, the next step in the synthesis is to conduct an amidation reaction between the oxy ester intermediate of Formula IV in which R₁, R₂, R₃ and p are as above, and an alkylene diamine as described by Formula V in which m is represented by 1 or 2. The product of this amidation reaction then cyclizes in-situ thereby producing the desired compound of Formula I. The combination of the amidation and cyclization serves to place the imidazoline or the tetrahydro-pyrimidine moiety on the oxy ester intermediate of Formula IV, thereby producing the desired compound of Formula I. 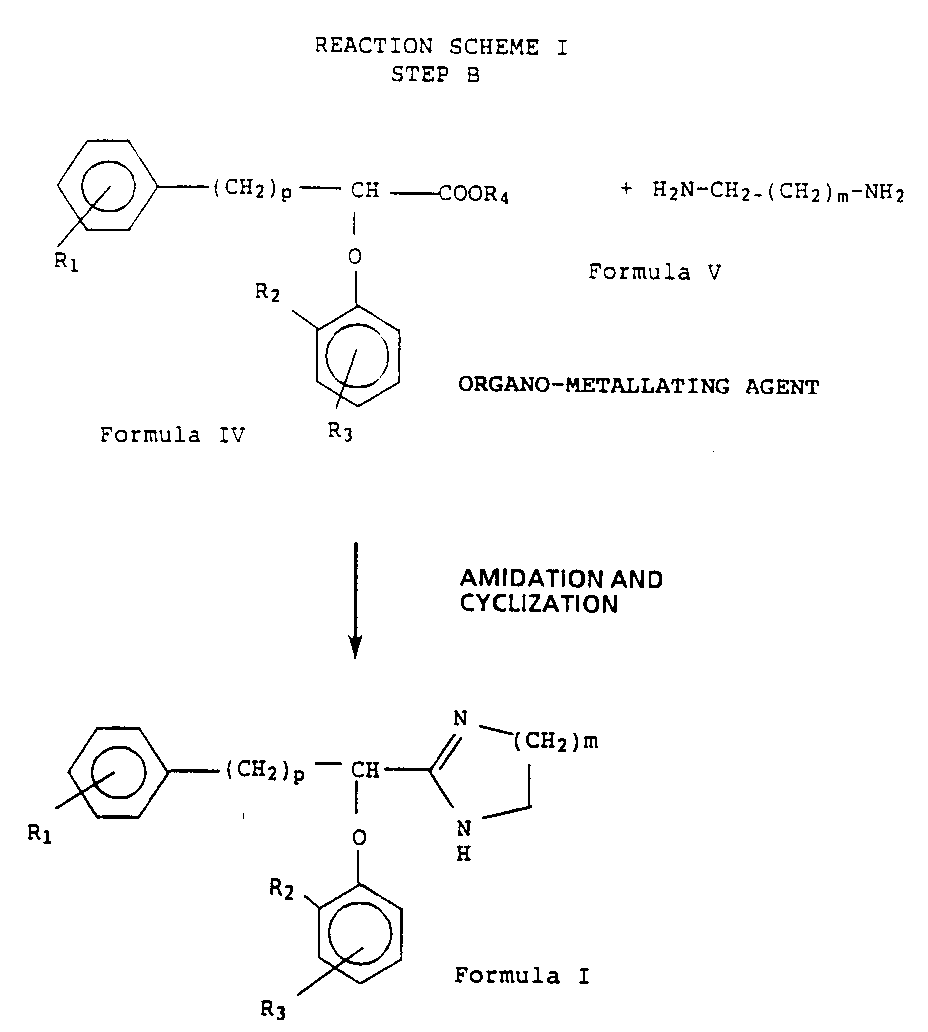
The appropriate alkylene diamine is one in which m is represented by 1, if the desired compound of Formula I is to contain an imidazoline ring and in which m is represented by 2, if a tetrahydro-pyrimidine ring is desired.

This amidation reaction can be conducted using techniques well known in the art. Approximately equimolar amounts of the oxy ester intermediate and the alkylene diamine are contacted in an organic solvent such as toluene. A suitable organo-metallating agent, such as, for example, Al(CH₃)₃ is added to the reaction mixture and the reactants are heated to reflux for a period of time ranging from about 3 to 8 hours. Typically from about 1 to about 1.5 equivalents of the organo-metallating agent is utilized. The product of the amidation reaction will cyclize in-situ during this refluxing period, thereby producing the desired compound of formula I.

The resulting compound of Formula I can be recovered and purified by techniques known in the art. For example, the compounds can be recovered from the reaction zone by either concentration or extraction. The compounds of Formula I can then be purified by chromatographic techniques known in the art such as silica gel chromatography. Alternatively, they can also be purified by recrystallization from a solvent system such as hexane or cyclohexane.

Methods for producing the phenols of Formula III, the bromo esters of Formula II, and the alkylene diamines of Formula V are known in the art.

Alternatively the oxy intermediates of Formula IV can be prepared as disclosed below in Reaction Scheme II: 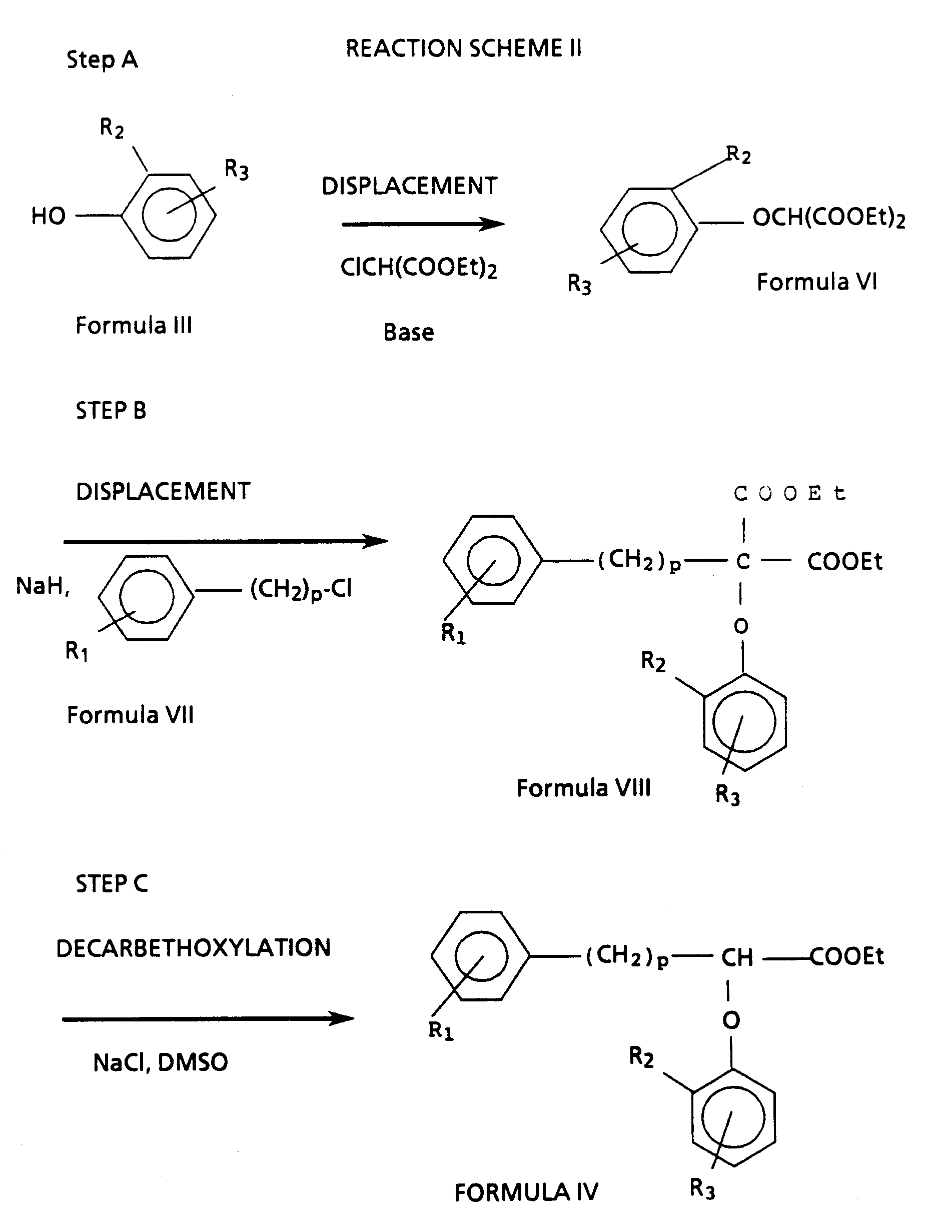
As is depicted in Reaction Scheme II, the initial step is to carry out a displacement reaction between a phenol as previously described by Formula III in which R₂ and R₃ are as defined above and diethyl chloromalonate. This produces the phenoxy derivative of Formula VI in which R₂ and R₃ are as in Formula I. In Step B the phenoxy derivative is subjected to a displacement reaction with a chloroalkylphenyl derivative as described by Formula VII in which R₁ and p are as in Formula I, which produces the intermediate of Formula VIII. This intermediate is then subjected to a decarbethoxylation reaction which produces the oxy ester of Formula IV in which R₄ is an ethyl moiety as depicted. The desired compound of Formula I can then be produced by the amidation and cyclization reaction depicted in Step B of Reaction Scheme I.

The proper starting material to utilize in the displacement reaction of Step A is a phenol derivative in which R₂ and R₃ are represented by the same substituents as is desired in the final product of Formula I. The displacement reaction of Step A can be carried out using techniqes known in the art. Typically approximately equivalent amounts of the phenol derivative and the diethyl chloromalonate are contacted in the presence of an excess of a base such as potassium carbonate. The reactants are heated to reflux in an organic solvent such as acetone for a period of time ranging from 10 to 48 hours. The desired phenoxy derivatives of Formula VI can be recovered by filtration and purified by distillation as is known in the art.

The displacement reaction of Step B is typically carried out in the following mannner. The phenoxy derivative of Formula VI is contacted with 1.1 equivalents of sodium hydride in excess dimethylformamide at a temperature range of from 5 to 10°C for a period of time from 0.5 to 1 hour. An equivalent amount of the chloroalkylphenyl derivative of Formula VII is then added to the reaction and the reactants are heated to a temperature range of from 55 to 60°C for a period of time from 2 to 6 hours. The desired intermediates of Formula VIII can be recovered by extraction and purified by distillation as is known in the art.

The decarbethoxylation of Step C is carried out by contacting the intermediate of Formula VIII with approximately 2 equivalents of water, 1 equivalent of NaCl, and an excess of DMSO. The reactants are heated to reflux under a nitrogen atmosphere for a period of time ranging from 2 to 8 hours. The desired oxy ester of Formula IV can be recovered by extraction and purified by distillation as is known in the art.

The compounds of Formula I are serotonin 5HT_{1A} agonists. They also have an affinity for the α-2 receptor. They are useful in the treatment of anxiety, depression, and hypertension.

The affinity of the compounds for the α-2 receptor can be demonstrated by receptor binding assay procedures which are known in the art, such as that described by Perry et al. in the European Journal of Pharmacology, Volume 76, pages 461-464 (1981). The affinity of the compounds for the 5HT_{1A} receptor can be demonstrated by receptor binding assay procedures such as described by Gozlan et al. in Nature, Volume 305, at pages 140-142 (1983). The procedure of Sleight et al., as disclosed in the European Journal of Pharmacology, Volume 154, pages 255-261 (1988) can be utilized to show that this affinity results in an agonistic effect upon the receptor.

It has been reported that 5HT_{1A} agonists are effective in the treatment of depression. The 5HT_{1A} agonist, 8-hydroxy-2-(di-N-propylamino) tetralin (8-OH-DPAT) was shown to be effective in rodent models for depression. European Journal of Pharmacology, Vol 144., pages 223-229 (1987) Ceroo et al. and European Journal of Pharmacology, Vol 158, pages 53-59 (1988) Ceroo et al. Since the compounds of the instant invention are 5HT_{1A} agonists, they will be useful in the treatment of depression.

In order to exhibit an anti-depressant effect, it is necessary that the compounds be administered to the patient in an effective amount. The dosage range at which these compounds exhibit this anti-depressant effect can vary widely depending upon the severity of the patient's depression, the particular compound being administered, the route of administration, the co-administration of other therapeutic agents, and the presence of other underlying disease states. Typically, the compounds will be administered at a dosage range of from 0.1 mg/kg/day to about 100 mg/kg/day. Repetitive daily administration may be desirable and will vary with the conditions described above. However, the compounds are typically administered from 1 to 4 times daily.

As used in this application, the term "depression" should be construed as encompassing those conditions which the medical profession have referred to as major depression, endogenous depression, psychotic depression, involutional depression, involutional melancholia, etc. These conditions are used to describe a condition in which patients typically experience intense sadness and despair, mental slowing, loss of concentration, pessimistic worry, despair, and agitation. The patients often experience physical complaints such as insomnia, anorexia, decreased energy, decreased libido, etc.

The compounds of Formula I will elevate the patient's mood if they are suffering from depression and either relieve or alleviate the physical complaints which the patient is experiencing.

As noted above, the compounds of Formula I are serotonin 5HT_{1A} agonists. Compounds producing this effect at the 5HT_{1A} receptor have also been found to exhibit anxiolytic properties. European Journal of Pharmocology, Vol. 88, pages 137-138 (1983) Gloser et al. and Drugs of the Future, Vol. 13, pages 429-439 (1988) Glaseat. A 5HT_{1A} agonist known as buspirone is currently being marketed as an anxiolytic agent. Since the compounds of the instant invention are 5HT_{1A} agonists, they-will be useful in the treatment of anxiety. The anxiolytic properties of these compounds can also be demonstrated by their ability to block distress vocalizations in rat pups. This test is based upon the phenomenon that when a rat pup is removed from its litter, it will emit an ultrasonic vocalization. It was discovered that anxiolytic agents block these vocalizations. The testing methods have been described by Gardner, C.R., Distress vocalization in rat pups: a simple screening method for anxiolytic drugs. J. Pharmacol. Methods, 14: 181-187 (1985) and Insel et al., Rat pup ultrasonic isolation calls: Possible mediation by the benzodiapine receptor complex, Pharmacol. Biochem. Behav., 24: 1263-1267 (1986).

As used in this application, the term "anxiety" refers to:
The unpleasant emotional state consisting of psychophysiological responses to anticipation of unreal or imagined danger, ostensibly resulting from unrecognized intrapsychic conflict. Physiological concomitants include increased heart rate, altered respiration rate, sweating, trembling, weakness, and fatigue; psychological concomitants include feelings of impending danger, powerlessness, apprehension, and tension.

In order to exhibit this anxiolytic effect, it is necessary that the compounds be administered to the patient in an effective amount. The dosage range at which these compounds exhibit this anxiolytic effect can vary widely depending upon the severity of the patient's anxiety, the particular compound being administered, the route of administration, the co-administration of other therapeutic agents, and the presence of other underlying disease states. Typically, the compounds will be administered at a dosage range of from 0.1 mg/kg/day to 100 mg/kg/day. Repetitive daily administration may be desirable and will vary with the conditions described above. However, the compounds are typically administered from 1 to 4 times daily.

Serotonin 5HT_{1A} agonists have also been shown to produce a hypotensive effect. For example, the 5HT_{1A} agonists, 8-OH-DPAT and Flesinoxan, have been shown to lower blood pressure. Dreteler et al., European Journal of Pharmacology, 180, pages 339-349 (199°c). Since the compounds of Formula I are 5HT_{1A} agonists, they will be useful in the treatment of hypertension. The anti-hypertensive properties of the compounds can be demonstrated by animal models known in the art such as the spontaneously hypertensive rat. This protocol has been described by Dage et al., Journal of Cardiovascular Pharmacology 3: 299-315 (1981).

In order to exhibit an antihypertensive effect, it is necessary that the compounds be administered to the patient in an effective amount. The dosage range at which these compounds exhibit this effect can vary widely depending upon the severity of the patient's condition, the particular compound being administered, the route of administration, the co-administration of other therapeutic agents, and the presence of other underlying disease states. Typically, the compounds will be administered at a dosage range of from 0.01 mg/kg/day to 100 mg/kg/day. Repetitive daily administration may be desirable and will vary with the conditions described above. However, the compounds are typically administered from 1 to 4 times daily.

The compounds of the present invention may be administered by a variety of routes. They are effective if administered orally. The compounds may also be administered parenterally (i.e. subcutaneously, intravenously, intramuscularly, or intraperitoneally).

As used in this application:
a) the term "patient" refers to warm blooded animals such as, for example, guinea pigs, mice, rats, cats, rabbits, dogs, monkeys, chimpanzees, and humans;
b) the term "treat" refers to the ability of the compounds to either relieve, alleviate, or slow the progression of the patient's disease.

Pharmaceutical compositions can be manufactured utilizing techniques known in the art. Typically an anti-depressant or anxiolytic amount of the compound will be admixed with a pharmaceutically acceptable carrier.

For oral administration, the compounds can be formulated into solid or liquid preparations such as capsules, pills, tablets, lozenges, melts, powders, suspensions, or emulsions. Solid unit dosage forms can be capsules of the ordinary gelatin type containing, for example, surfactants, lubricants and inert fillers such as lactose, sucrose, and cornstarch or they can be sustained release preparations. In another embodiment, the compounds of Formula I can be tableted with conventional tablet bases such as lactose, sucrose, and cornstarch in combination with binders, such as acacia, cornstarch, or gelatin, disintegrating agents such as potato starch or alginic acid, and a lubricant such as stearic acid or magnesium stearate. Liquid preparations are prepared by dissolving the active ingredient in an aqueous or non-aqueous pharmaceutically acceptable solvent which may also contain suspending agents, sweetening agents, flavoring agents, and preservative agents as are known in the art.

For parenteral administration the compounds may be dissolved in a physiologically acceptable pharmaceutical carrier and administered as either a solution or a suspension. Illustrative of suitable pharmaceutical carriers are water, saline, dextrose solutions, fructose solutions, ethanol, or oils of animal, vegetative, or synthetic origin. The pharmaceutical carrier may also contain preservatives, buffers, etc., as are known in the art.

The compounds of Formula I may also be admixed with any inert carrier and utilized in laboratory assays in order to determine the concentration of the compounds within the serum, urine, etc., of the patient as is known in the art.

### Example 1

The purpose of this example is to demonstrate the alkylation reaction between a bromo ester as described by Formula II and an alcohol as described by Formula III which is described in Step A of Reaction Scheme I.

### Ethyl α-(2-Methoxyphenoxy)-phenylacetate

A mixture of 130 g (1.05 M) guaiacol, 243, g (1 M) ethyl α-bromophenylacetate, 150 g of potassium carbonate and 1.5 L of acetone was refluxed for 96 hours, cooled and filtered. The solvent was removed at reduced pressure and the residue dissolved in toluene. After washing with dilute sodium hydroxide, then water, the solvent was removed and the residue distilled. The fraction boiling at 158-163°C/0.4 mm was collected, weight 263 g.

| | | |
|---|---|---|
| Anal., Calcd. for C₁₇H₁₈O₄: | C=71.21; | H= 6.34 |
| Fd.: | C=71.26; | H= 6.49 |

Similarly prepared were:
Ethyl α-phenoxyphenylacetate (literature compound)
Ethyl 2-(2-ethoxyphenoxy)phenylacetate
Boiling Point 130-138°C/0.05 mm

| | | |
|---|---|---|
| Anal., Calcd. for C₁₈H₂₀O₄: | C=71.98; | H=6.71 |
| Fd.: | C=71.71; | H=6.73 |

Ethyl 2-(2-methoxyphenoxy)-3-phenylpropionate
Boiling Point 130-40°C/0.3 mm

| | | |
|---|---|---|
| Anal., Calcd. for C₁₈H₂₀O₄: | C=71.98; | H=6.71 |
| Fd.: | C=72.32; | H=6.64 |

Ethyl 2-(2,3-dimethoxyphenoxy)-3-phenylpropionate
Boiling Point 150-58°C/0.3 mm

| | | |
|---|---|---|
| Anal., Calcd. for C₁₉H₂₂O₅: | C=69.07; | H=6.71 |
| Fd.: | C=69.16; | H=6.69 |

Ethyl 2-(2-ethoxyphenoxy)-3-phenylpropionate
Boiling Point 130-35°C/0.25 mm

| | | |
|---|---|---|
| Anal., Calcd. for C₁₉H₂₂O₄: | C=72.59; | H=7.05 |
| Fd.: | C=72.39; | H=6.98 |

Ethyl 2-(2-methoxyphenoxy)-4-phenylbutyrate
Boiling Point 143-48°C/0.1 mm

| | | |
|---|---|---|
| Anal. Calcd. for C₁₉H₂₂O₄: | C=72.59; | H=7.05 |
| Fd.: | C=72.50; | H=7.05 |

Ethyl 2-(2-ethoxyphenoxy)-4-phenylbutyrate
Boiling Point 175-82°C/0.5 mm

| | | |
|---|---|---|
| Anal. Calcd. for C₂₀H₂₄O₂: | C=73.14; | H=7.37 |
| Fd.: | C=72.75; | H=7.25 |

Ethyl 2-(2,6-Dimethoxyphenoxy)phenylacetate
Boiling Point 165-75°C/0.3 mm

| | | |
|---|---|---|
| Anal. Calcd. for C₁₈H₂₀O₅: | C=68.34; | H=6.37 |
| Fd.: | C=68.36; | H=6.42 |

Using the methods taught above but substituting the appropriate starting material, the following compounds can be prepared.
Ethyl 2-(2-methoxyphenoxy)-4-phenylbutyrate
Ethyl 2-(2-ethoxyphenoxy)-5-phenylvalerate
Ethyl 2-(2-methoxyphenoxy)-2-(3,4-dichlorophenyl)acetate
Methyl 2-(2,5-dimethoxyphenoxy)-4-phenylbutyrate
Ethyl 2-(2-isopropylthiophenoxy)-3-phenylpropionate
Ethyl 2-(4-chlorophenoxy)phenylacetate.

### EXAMPLE 2

The purpose of this example is to demonstrate a displacement reaction depicted in Step A of Reaction Scheme II.

### A) Diethyl 2-(isopropoxy)phenoxymalonate

A mixture of 25 g (0.16 M) diethyl chloromalonate, 25 g potassium carbonate and 320 ml of acetone was refluxed for 24 hours, cooled and filtered. The solvent was removed from the filtrate and the residue taken up in ether, washed with water and dried over sodium sulfate. Removal of the solvent and distillation gave 36.3 g boiling at 145-148°C/0.2 mm.

| | | |
|---|---|---|
| Anal. Calcd. for C₁₆H₂₂O₆: | C=61.92; | H=7.15. |
| Fd: | C=61.44; | H=7.06. |

Similarly prepared were:
Diethyl 2-ethoxyphenoxymalonate
Boiling point 155-162°C/0.3 mm

| | | |
|---|---|---|
| Anal. Calcd. for C₁₅H₂₀O₆: | C=60.80; | H=6.80 |
| Fd: | C=60.73; | H=6.73 |

Diethyl 2-methylthiophenoxymalonate
Boiling point 177-182°C/0.3 mm

| | | |
|---|---|---|
| Anal. Calcd. for C₂₁H₂₄O₅S: | C=64.92; | H=6.23 |
| Fd: | C=64.75; | H=6.24 |

This example demonstrates the displacement reaction of Step B in Reaction Scheme II.

### B) Diethyl α-benzyl-α-[2-(isopropoxy)phenoxy]malonate

To an ice-cooled suspension of sodium hydride (from 2.2 g of a 60% mixture with oil) is added 100 ml of dimethylformamide, a solution of 16.1 g (0.05 M) or diethyl 2-(isopropoxy)phenoxymalonate in 25 ml of dimethylformamide was added dropwise. After stirring 20 minutes at room temperature a solution of 7.0 g (0.055 M) of benzyl chloride in 10 ml of dimethylformamide was added all at once and the mixture was heated in an oil bath at 55-60°C for 2 hours. The mixture was cooled in ice and excess sodium hydride was decomposed with acetic acid. The reaction mixture was diluted with water and extracted with ethyl acetate. The ethyl acetate was removed from the extracts and the residue was shaken with a mixture of acetonitrile and pentane. Concentration of the acetonitrile layer and distillation gave 17.5 g, b.p. 175-185°C/0.2 mm.

| | | |
|---|---|---|
| Anal. Calcd. for C₂₃H₂₈O₆: | C=68.98; | H=7.05 |
| Fd: | C=68.83; | H=7.05. |

Similarly prepared were:
Diethyl α-(4-fluorobenzyl)-α-(2-ethoxyphenoxy)malonate
Boiling point 180-183°C/0.3 mm

| | | |
|---|---|---|
| Anal. Calcd. for C₂₂H₂₅FO₆: | C=65.33; | H=6.23 |
| Fd: | C=65.23; | H=6.45 |

Diethyl α-(4-t-butyl-2,6-dimethylbenzyl)-α-(2-ethoxyphenoxy)malonate
Boiling point 178-187°C/0.2 mm

| | | |
|---|---|---|
| Anal. Calcd. for C₂₇H₃₈O₆: | C=70.71; | H=8.35 |
| Fd: | C=70.57; | H=8.62 |

This example demonstrates the decarbethoxylation reaction of Step C in Reaction Scheme II.

### C) Ethyl 2-(2-isopropoxyphenoxy)-3-phenyl propionate

A mixture of 17.2 g (0.043 M) of diethyl α-benzyl-2-isopropoxyphenoxymalonate, 2.5 g (0.04 M) sodium chloride, 1.55 g (0.085 M) water and 120 ml of dimethylsulfoxide was refluxed under nitrogen for 6 hours, cooled and diluted with water. The mixture was extracted with two 500 ml portions of 4:1 pentane-ether and the extracts dried over magnesium sulfate. Removal of the solvent and distillation gave 10.6 g, b.p. 138-143°C/0.3 mm.

| | | |
|---|---|---|
| Anal. Calcd. for C₂₀H₂₄O₄: | C=73.14; | H=7.37 |
| Fd: | C=72.54; | H=7.42 |

Similarly prepared were:
Ethyl 2-(ethoxyphenoxy)-3-(4-fluorophenyl)propionate
Boiling Point 137-143°C/0.3 mm

| | | |
|---|---|---|
| Anal. Calcd. for C₁₉H₂₁FO₄: | C=67.48; | H=6.61 |
| Fd: | C=68.05; | H=6.39 |

Ethyl 2-(ethoxyphenoxy)-3-(4-methoxyphenyl)propionate
Boiling Point 175-178°C/0.4 mm

| | | |
|---|---|---|
| Anal. Calcd.for C₂₀H₂₄O₅: | C=69.78; | H=7.02 |
| Fd: | C=69.67; | H=7.11 |

### Example 3

The purpose of this example is to demonstrate the amidation and cyclization reaction which is described in Step B of Reaction Scheme I.

### 2-[1-(2-ethoxyphenoxy)-2-phenylethyl]imidazoline

A mixture of 6.28 g (0.02 M) of ethyl 2-(2-ethoxyphenoxy)-3-phenylpropionate, 1.93 g (0.033 M) ethylenediamine and 150 ml of dry toluene was stirred at room temperature and 17 ml of a 2 M solution of trimethylaluminum in toluene was added dropwise. After stirring 10 minutes, the mixture was heated at reflux for 3 hours, then cooled in an ice bath. Water (10 ml) was added followed by 20 ml methanol and the mixture heated on a steam bath for 0.5 hours. Solids were filtered and the solvent evaporated. The solid residue was recrystallized from cyclohexane to give 3.53 g, melting point 95-97°C.

| | | | |
|---|---|---|---|
| Anal., Calcd. for C₁₉H₂₂N₂O₂: | C=73.52; | H=7.14; | N=9.03 |
| Fd.: | C=73.65; | H=7.21; | N=8.93 |

Similarly prepared were:
2-[α-(2-methoxyphenoxy)]benzylimidazoline
Melting Point 123-25°C

| | | | |
|---|---|---|---|
| Anal. Calcd. for C₁₇H₁₈N₂O₂: | C=72.32; | H=6.43; | N=9.92 |
| Fd.: | C=72.08; | H=6.45; | N=9.79 |

2-[α-(2-Ethoxyphenoxy)]benzylimidazoline
Melting Point 116-18°C

| | | | |
|---|---|---|---|
| Anal. Calcd. for C₁₈H₂₀N₂O₂: | C=72.94; | H=6.80; | N=9.45 |
| Fd.: | C=72.86; | H=6.84; | N=9.44 |

2-[1-(2-methoxyphenoxy)-2-phenylethyl]imidazoline
Melting Point 97-100°C

| | | | |
|---|---|---|---|
| Anal. Calcd. for C₁₈H₂₀N₂O₂: | C=72.94; | H=6.80; | N=9.45 |
| Fd.: | C=72.48; | H=6.80; | N=9.58 |

2-[1-(2-ethoxyphenoxy)-2-phenylethyl]imidazoline
Melting Point 95-97°C

| | | | |
|---|---|---|---|
| Anal. Calcd. for C₁₉H₂₂N₂O₂: | C=73.52; | H=7.14; | N=9.03 |
| Fd.: | C=73.65; | H=7.21; | N=8.93 |

2-[1-(2,3-dimethoxyphenoxy)-2-phenylethyl]imidazoline
Melting Point 86-87°C

| | | | |
|---|---|---|---|
| Anal. Calcd. for C₁₉H₂₂N₂O₃: | C=69.92; | H=6.79; | N=8.58 |
| Fd.: | C=69.72; | H=6.84; | N=8.51 |

2-[1-(2-methoxyphenoxy)-3-phenylpropyl]imidazoline
Melting point 117-118°C

| | | | |
|---|---|---|---|
| Anal. Calcd. for C₁₉H₂₂N₂O₂: | C=73.52; | H=7.14; | N=9.03 |
| Fd.: | C=73.53; | H=7.23; | N=8.96 |

2-[1-(2-ethoxyphenoxy)-3-phenylpropyl]imidazoline
Melting Point 74-77°C

| | | | |
|---|---|---|---|
| Anal. Calcd. for C₂₀H₂₄N₂O₂: | C=74.04; | H=7.46; | N=8.64 |
| Fd.: | C=73.84; | H=7.52; | N=8.49 |

2-[1-(2-Ethoxyphenoxy)-2-phenylethyl)-1,2,3,4-tetrahydropyrimidine
Melting point 98-100°C

| | | | |
|---|---|---|---|
| Anal. Calcd. for C₂₀H₂₄N₂O₂: | C=74.04; | H=7.46; | N=8.64 |
| Fd: | C=73.90; | H=7.52; | N=8.44 |

2-[1-(2-Ethoxyphenoxy)-(4-fluorophenyl)ethyl]imidazoline
Melting point 103-105°C

| | | | |
|---|---|---|---|
| Anal. Calcd. for C₁₉H₂₁FN₂O₂: | C=69.49; | H=6.45; | N=8.53 |
| Fd: | C=69.23; | H=6.48; | N=8.70 |

2-[1-(2-Ethoxyphenoxy)-(4-methoxyphenyl)ethyl]imidazoline
Melting point 89-91°C

| | | | |
|---|---|---|---|
| Anal. Calcd. for C₂₀H₂₄FN₂O₃: | C=70.56; | H=7.11; | N=8.23 |
| Fd: | C=70.61; | H=7.24; | N=8.25 |

2-[1-(2-Ethoxyphenoxy)-(4-t-butyl-2,6-dimethylphenyl)ethyl]imidazoline oxalate
Melting point 205-207°C

| | | | |
|---|---|---|---|
| Anal. Calcd. for C₂₅H₃₄FN₂O₂·C₂H₂O₄: | C=66.92; | H=7.49; | N=5.78 |
| Fd: | C=66.95; | H=7.71; | N=5.67 |

2-[1-(2-Isopropoxypheonxy)-2-phenylethyl]imidazoline
Melting point 81-83°C

| | | | |
|---|---|---|---|
| Anal. Calcd. for C₂₀H₂₄N₂O₂: | C=74.27; | H=7.17; | N=8.66 |
| Fd: | C=73.87; | H=7.50; | N=8.38 |

2-[α-(2-Cyclopentyloxy)-2-phenylethyl]imidazoline
Melting point 110-112°C

| | | | |
|---|---|---|---|
| Anal. Calcd. for C₂₂H₂₆N₂O₂: | C=75.40; | H=7.48; | N=7.99 |
| Fd: | C=75.10; | H=7.46; | N=7.73 |

2-[1-(2-Ethoxy-5-methoxyphenoxy)-2-phenylethyl]imidazoline oxalate
Melting point 132-134°C

| | | | |
|---|---|---|---|
| Anal. Calcd. for C₂₀H₂₄N₂O₃·C₂H₂O₄: | C=61.38; | H=6.09; | N=6.51 |
| Fd: | C=61.46; | H=6.18; | N=6.48 |

2-[1-(2,6-Dimethylphenoxy)-2-phenylethyl]imidazoline
Melting Point 119-122°C

| | | | |
|---|---|---|---|
| Anal. Calcd. for C₁₉H₂₂N₂O: | C=77.51; | H=7.53; | N=9.52 |
| Fd: | C=77.40; | H=7.68; | N=9.29 |

2-[α-(2,3-Dimethoxyphenoxy)benzyl]imidazoline
Melting point 86-87°C

| | | | |
|---|---|---|---|
| Anal. Calcd. for C₁₉H₂₂N₂O₃: | C=69.92; | H=6.79; | N=8.58 |
| Fd: | C=69.72 | H=6.84; | N=8.51 |

Using the methods taught above,but substituting the appropriate starting material, the following compounds may be prepared.
2-[1-(2-methoxyphenoxy)-2-phenylethyl]imidazoline
2-[1-(2-methylphenoxy)-2-(4-chlorophenyl)ethyl]imidazoline
2-[1-(2-fluoro-4-methoxyphenoxy)-3-phenylpropyl]imidazoline
2-[2-(4-methoxyphenyl)-1-(2-methoxyphenoxy)ethyl]imidazoline
2-[3-(4-chlorophenyl)-1-(2-methoxyphenoxy)propyl]-1,2,3,4-tetrahydropyrimidine
2-[1-(2-methylthiophenoxy)-2-phenylethyl]-imidazoline
Melting point 112-114°C

| | | | |
|---|---|---|---|
| Anal. Calcd. for C₁₈H₂₀N₂O₅ | C=69.19; | H=6.45; | N=8.97 |
| Fd: | C=69.20; | H=6.58; | N=8.96 |

## Claims (Claims for the following Contracting State(s): AT, BE, CH, DE, DK, FR, GB, IT, LI, LU, NL, SE)

1. A compound of the formula: 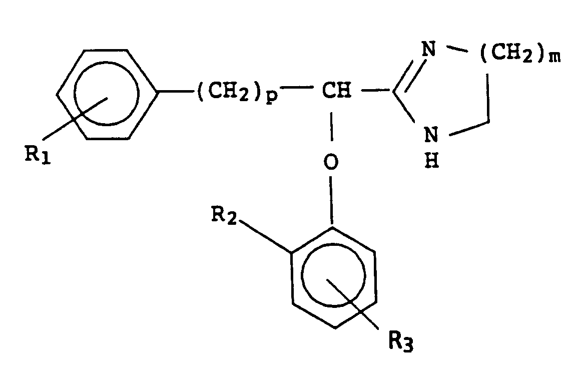 in which R₁ is represented by hydrogen, halogen, C₁₋₄ alkyl, C₁₋₄ alkoxy, OH, CF₃, CN, methylenedioxy, ethylenedioxy, C₃₋₅ methylene, 2,3-benzo, 3,4-benzo, alkylthio, alkylsulfonyl, alkylsulfinyl, C₃₋₆ cycloalkyl, C₅₋₈ cycloalkoxy or C₅₋₈ cycloalkylthio, there can be up to 5 monovalent substituents occurring on the phenyl ring which may be the same or different if R₁ is to be represented by a divalent substituent, there can be one of these substituents bonded to the phenyl ring which may optionally be substituted with one other non-hydrogen monovalent substituent; R₂ is represented by halogen, C₁₋₄ alkyl, C₁₋₄ alkoxy, OH, CF₃, CN, alkylthio, alkylsulfinyl, C₃₋₆ cycloalkyl, C₅₋₈ cycloalkoxy or C₅₋₈ cycloalkylthio; R₃ is represented by hydrogen, halogen, C₁₋₄ alkyl, C₁₋₄ alkoxy, OH, CF₃, CN, methylenedioxy, ethylenedioxy, C₃₋₅ methylene, 2,3-benzo, 3,4-benzo, 4,5-benzo, 5,6-benzo, alkylthio, alkylsulfonyl, alkylsulfinyl, C₃₋₆ cycloalkyl, C₅₋₈ cycloalkoxy or C₅₋₈ cycloalkylthio, R₃ may represent up to 4 monovalent substituents which may be the same or different or a single divalent substituent; m is represented by the integer 1 or 2; p is represented by an integer from 0-4; and the pharmaceutically acceptable addition salts thereof,
excluding the compound 1,4,5,6-tetrahydro-2-[α-(o-tolyloxy)benzyl]-pyrimidine hydrochloride.

2. A compound according to claim 1 wherein m is represented by 1.

3. A compound according to claim 1 wherein m is represented by 2.

4. A compound according to any of claims 1 to 3 wherein p is represented by 1.

5. A compound according to any of claims 1 to 4 wherein R₂ is alkoxy.

6. A compound according to any of claims 1 to 4 wherein R₂ is ethoxy.

7. A compound according to any of claims 1 to 6 wherein R₃ is hydrogen.

8. A process for preparing a compound of the formula I as defined in claim 1, comprising:
A) producing an oxy ester as described by Formula IV: 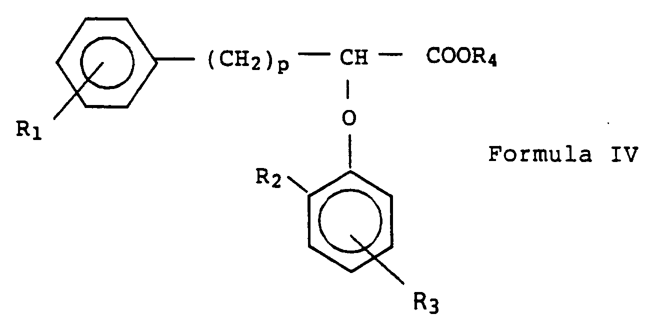 in which R₁, p, R₂, and R₃ are as above and R₄ is a C₁₋₄ alkyl by either Method #1 or #2:
1) carrying out an alkylation reaction between a bromo ester as described by Formula II: 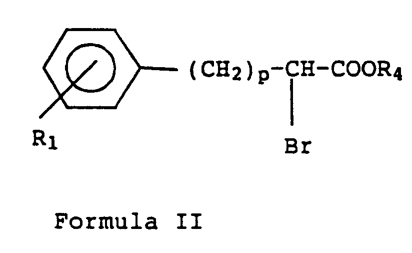 in which p and R₁ are as above and R₄ is a C₁₋₄ alkyl, and a phenol derivative as described by Formula III: 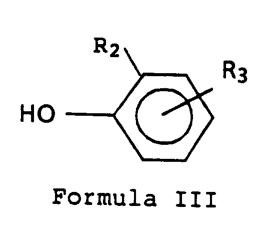 in which R₂ and R₃ are as above,
or
2)
a) subjecting a phenol derivative as described by Formula III: 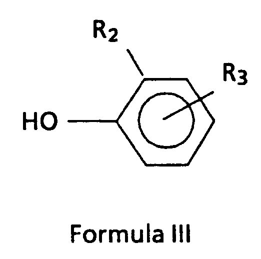 in which R₂ and R₃ are as above, to a displacement reaction with diethyl chloromalonate thereby producing the phenoxy derivative of Formula VI: 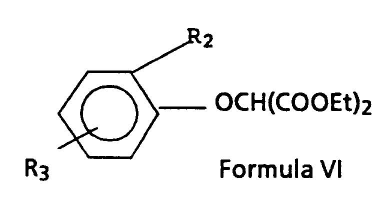 in which R₂ and R₃ are as above,
b) subjecting the resulting phenoxy derivative to a displacement reaction with a chloroalkylphenyl derivative as described by Formula VII: 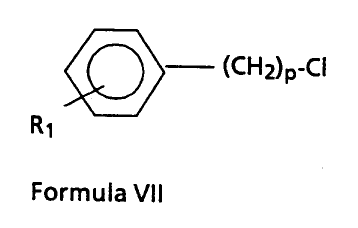 in which R₁ and p are as above, which produces the intermediate of Formula VIII: 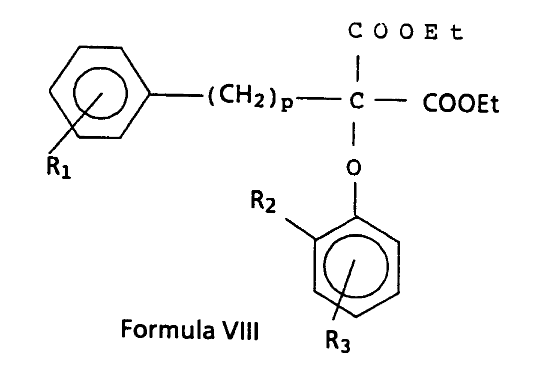 in which R₁, R₂, R₃, and p are as above,
and
c) subjecting this intermediate to a decarbethoxylation reaction which produces the desired oxy ester of Formula IV in which R₄ is ethyl,
and
B) subjecting the resulting oxy ester of Formula IV to an amidation and cyclization with a diamine as depicted by Formula V:
H₂N-CH₂-(CH₂)ₘ-NH₂ Formula V
in which m is as above.

9. A compound according to any of claims 1 to 7 for use as a therapeutically active agent.

10. A compound according to claim 9 for the treatment of depression.

11. A compound according to claim 9 for the treatment of anxiety.

12. A compound according to claim 9 for the treatment of hypertension.

13. A pharmaceutical composition comprising a compound according to any of claims 1 to 7 and a physiologically acceptable carrier or diluent.

14. Use of 1,4,5,6-tetrahydro-2[α-(o-tolyloxy)benzyl]-pyrimidine hydrochloride for the preparation of a pharmaceutical composition useful in the treatment of depression.

15. Use of 1,4,5,6-tetrahydro-2[α-(o-tolyloxy)benzyl]-pyrimidine hydrochloride for the preparation of a pharmaceutical composition useful in the treatment of anxiety.

16. Use of 1,4,5,6-tetrahydro-2[α-(o-tolyloxy)benzyl]-pyrimidine hydrochloride for the preparation of a pharmaceutical composition useful in the treatment of hypertension.

## Claims (Claims for the following Contracting State(s): GR, ES)

1. A compound of the formula: 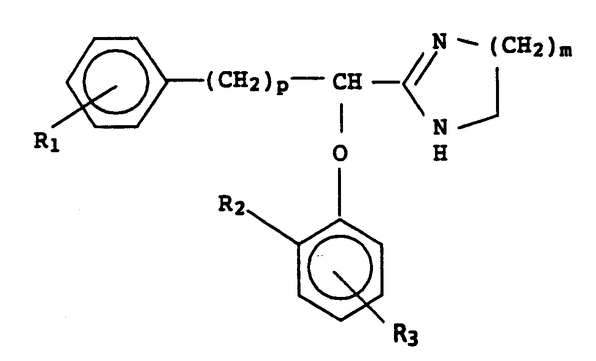 in which R₁ is represented by hydrogen, halogen, C₁₋₄ alkyl, C₁₋₄ alkoxy, OH, CF₃, CN, methylenedioxy, ethylenedioxy, C₃₋₅ methylene, 2,3-benzo, 3,4-benzo, alkylthio, alkylsulfonyl, alkylsulfinyl, C₃₋₆ cycloalkyl, C₅₋₈ cycloalkoxy or C₅₋₈ cycloalkylthio; there can be up to 5 monovalent substituents occurring on the phenyl ring which may be the same or different, if R₁ is to be represented by a divalent substituent, there can be one of these substituents bonded to the phenyl ring which may optionally be substituted with one other non-hydrogen monovalent substituent; R₂ is represented by halogen, C₁₋₄ alkyl, C₁₋₄ alkoxy, OH, CF₃, CN, alkylthio, alkylsulfinyl, C₃₋₆ cycloalkyl, C₅₋₈ cycloalkoxy or C₅₋₈ cycloalkylthio; R₃ is represented by hydrogen, halogen, C₁₋₄ alkyl, C₁₋₄ alkoxy, OH, CF₃, CN, methylenedioxy, ethylenedioxy, C₃₋₅ methylene, 2,3-benzo, 3,4-benzo, 4,5-benzo, 5,6-benzo, alkylthio, alkylsulfonyl, alkylsulfinyl, C₃₋₆ cycloalkyl, C₅₋₈ cycloalkoxy or C₅₋₈ cycloalkylthio; R₃ may represent up to 4 monovalent substituents which may be the same or different or a single divalent substituent; m is represented by the integer 1 or 2; p is represented by an integer from 0-4; and the pharmaceutically acceptable addition salts thereof,
excluding the compound 1,4,5,6-tetrahydro-2-[α-(o-tolyloxy)benzyl]-pyrimidine hydrochloride.

2. A compound according to claim 1 wherein m is represented by 1.

3. A compound according to claim 1 wherein m is represented by 2.

4. A compound according to any of claims 1 to 3 wherein p is represented by 1.

5. A compound according to any of claims 1 to 4 wherein R₂ is alkoxy.

6. A compound according to any of claims 1 to 4 wherein R₂ is ethoxy.

7. A compound according to any of claims 1 to 6 wherein R₃ is hydrogen.

8. A process for preparing a compound of the formula I as defined in claim 1, comprising:
A) producing an oxy ester as described by Formula IV: 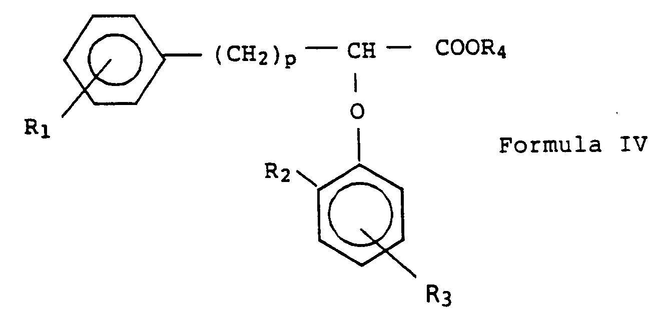 in which R₁, p, R₂, and R₃ are as above and R₄ is a C₁₋₄ alkyl by either Method #1 or #2:
1) carrying out an alkylation reaction between a bromo ester as described by Formula II: 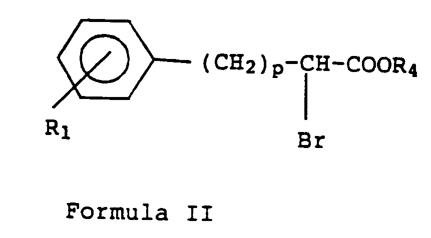 in which p and R₁ are as above and R₄ is a C₁₋₄ alkyl, and a phenol derivative as described by Formula III: 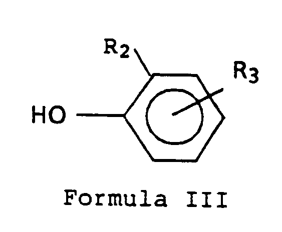 in which R₂ and R₃ are as above,
or
2)
a) subjecting a phenol derivative as described by Formula III: 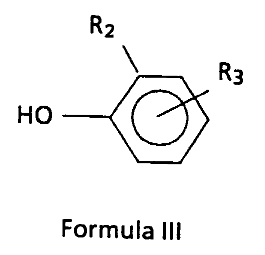 in which R₂ and R₃ are as above, to a displacement reaction with diethyl chloromalonate thereby producing the phenoxy derivative of Formula VI: 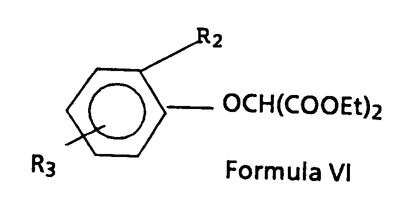 in which R₂ and R₃ are as above,
b) subjecting the resulting phenoxy derivative to a displacement reaction with a chloroalkylphenyl derivative as described by Formula VII: 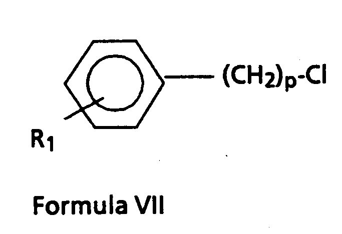 in which R₁ and p are as above, which produces the intermediate of Formula VIII: 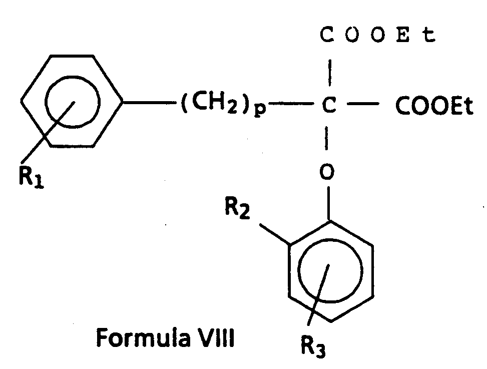 in which R₁, R₂, R₃, and p are as above,
and
c) subjecting this intermediate to a decarbethoxylation reaction which produces the desired oxy ester of Formula IV in which R₄ is ethyl,
and
B) subjecting the resulting oxy ester of Formula IV to an amidation and cyclization with a diamine as depicted by Formula V:
H₂N-CH₂-(CH₂)ₘ-NH₂ Formula V
in which m is as above.

9. A method for the preparation of a pharmaceutical composition comprising combining a compound as defined in any one of claims 1 to 7 with a pharmaceutically acceptable carrier.

10. A method according to claim 9 wherein the pharmaceutical composition prepared is for the treatment of depression.

11. A method according to claim 9 wherein the pharmaceutical composition prepared is for the treatment of anxiety.

12. A method according to claim 9 wherein the pharmaceutical composition prepared is for the treatment of hypertension.

## Patentansprüche (Patentansprüche für folgende(n) Vertragsstaat(en): AT, BE, CH, DE, DK, FR, GB, IT, LI, LU, NL, SE)

1. Verbindung der Formel: 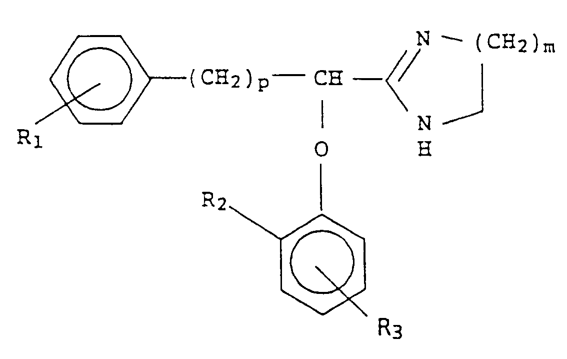 in der R₁ ein Wasserstoff- oder Halogenatom, einen C₁₋₄-Alkyl- oder C₁₋₄-Alkoxyrest, eine OH-, CF₃-, CN-, Methylendioxy- oder Ethylendioxygruppe, einen C₃₋₅-Methylenrest, eine 2,3-Benzo- oder 3,4-Benzogruppe, einen Alkylthio-, Alkylsulfonyl-, Alkylsulfinyl-, C₃₋₆-Cycloalkyl-, C₅₋₈-Cycloalkoxy- oder C₅₋₈-Cycloalkylthiorest darstellt, wobei bis zu 5 monovalente Substituenten am Phenylring auftreten können, die gleich oder verschieden sein können, und falls R₁ einen divalenten Substituenten darstellt, kann einer dieser Substituenten an den Phenylring gebunden sein, der gegebenenfalls mit einem weiteren monovalenten Nichtwasserstoffsubstituenten substituiert sein kann; R₂ ein Halogenatom, einen C₁₋₄-Alkyl- oder C₁₋₄-Alkoxyrest, eine OH-, CF₃- oder CN-Gruppe, einen Alkylthio-, Alkylsulfinyl-, C₃₋₆-Cycloalkyl-, C₅₋₈-Cycloalkoxy- oder C₅₋₈-Cycloalkylthiorest bedeutet; R₃ ein Wasserstoff- oder Halogenatom, einen C₁₋₄-Alkyl- oder C₁₋₄-Alkoxyrest, eine OH-, CF₃-, CN-, Methylendioxy- oder Ethylendioxygruppe, einen C₃₋₅-Methylenrest, eine 2,3-Benzo-, 3,4-Benzo-, 4,5-Benzo- oder 5,6-Benzogruppe, einen Alkylthio-, Alkylsulfonyl-, Alkylsulfinyl-, C₃₋₆-Cycloalkyl-, C₅₋₈-Cycloalkoxy- oder C₅₋₈-Cycloalkylthiorest darstellt, R₃ bis zu 4 monovalente Substituenten, die gleich oder verschieden sein können, oder einen einzigen divalenten Substituenten bedeuten kann; m die ganze Zahl 1 oder 2 darstellt; p eine ganze Zahl von 0 - 4 bedeutet; und die pharmazeutisch verträglichen Additionssalze davon,
wobei die Verbindung 1,4,5,6-Tetrahydro-2-[α-(o-tolyloxy)benzyl]-pyrimidinhydrochlorid ausgeschlossen ist.

2. Verbindung nach Anspruch 1, wobei m 1 bedeutet.

3. Verbindung nach Anspruch 1, wobei m 2 darstellt.

4. Verbindung nach einem der Ansprüche 1 bis 3, wobei p 1 bedeutet.

5. Verbindung nach einem der Ansprüche 1 bis 4, wobei R₂ einen Alkoxyrest darstellt.

6. Verbindung nach einem der Ansprüche 1 bis 4, wobei R₂ einen Ethoxyrest bedeutet.

7. Verbindung nach einem der Ansprüche 1 bis 6, wobei R₃ ein Wasserstoffatom darstellt.

8. Verfahren zur Herstellung einer Verbindung der Formel I, wie sie in Anspruch 1 definiert ist, umfassend:
A) Herstellung eines Oxyesters, wie er durch Formel IV beschrieben wird: 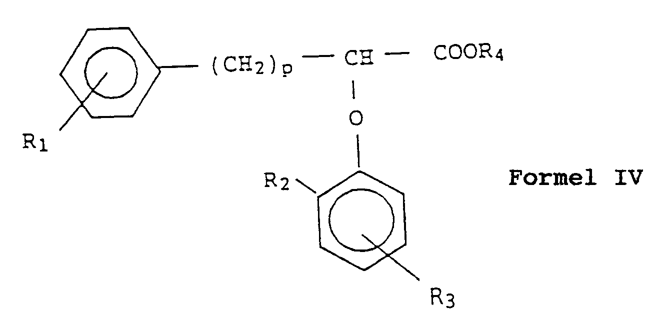 in der R₁, p, R₂ und R₃ wie vorstehend sind und R₄ einen C₁₋₄-Alkylrest darstellt, entweder durch Verfahren #1 oder #2:
1) Ausführen einer Alkylierungsreaktion zwischen einem Bromester, wie er durch Formel II beschrieben wird: 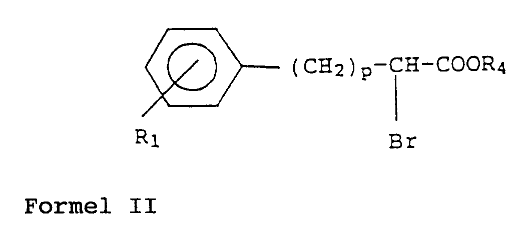 in der p und R₁ wie vorstehend sind und R₄ einen C₁₋₄-Alkylrest darstellt, und einem Phenolderivat, wie es durch Formel III beschrieben wird: 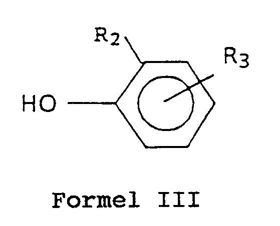 in der R₂ und R₃ wie vorstehend sind,
oder
2)
a) Unterwerfen eines Phenolderivats, wie es durch Formel III beschrieben wird: 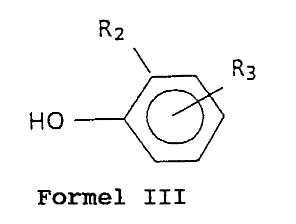 in der R₂ und R₃ wie vorstehend sind, einer Verdrängungsreaktion mit Chlormalonsäurediethylester, wobei das Phenoxyderivat der Formel VI erzeugt wird: 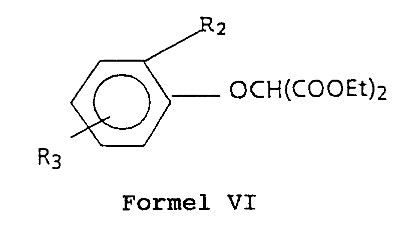 in der R₂ und R₃ wie vorstehend sind,
b) Unterwerfen des erhaltenen Phenoxyderivats einer Verdrängungsreaktion mit einem Chloralkylphenylderivat, wie es durch Formel VII beschrieben wird: 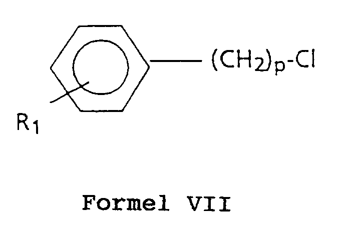 in der R₁ und p wie vorstehend sind, wodurch das Zwischenprodukt der Formel VIII erzeugt wird: 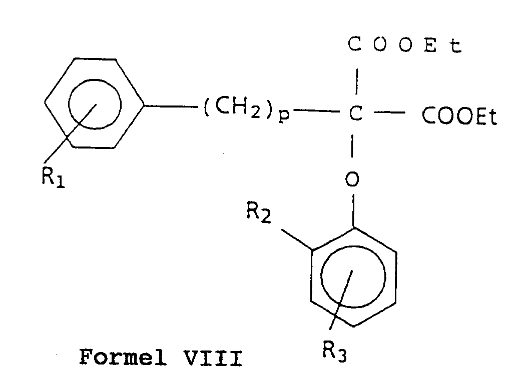 in der R₁, R₂, R₃ und p wie vorstehend sind,
und
c) Unterwerfen dieses Zwischenprodukts einer Decarbethoxylierungsreaktion, die den gewünschten Oxyester der Formel IV, in der R₄ eine Ethylgruppe bedeutet, erzeugt,
und
B) Unterwerfen des erhaltenen Oxyesters der Formel IV einer Amidierung und Cyclisierung mit einem Diamin, wie es durch Formel V veranschaulicht wird:
H₂N-CH₂-(CH₂)ₘ-NH₂ Formel V
in der m wie vorstehend ist.

9. Verbindung nach einem der Ansprüche 1 bis 7 zur Verwendung als therapeutischer Wirkstoff.

10. Verbindung nach Anspruch 9 zur Behandlung von Depressionen.

11. Verbindung nach Anspruch 9 zur Behandlung von Angst.

12. Verbindung nach Anspruch 9 zur Behandlung von Bluthochdruck.

13. Arzneimittel, das eine Verbindung nach einem der Ansprüche 1 bis 7 und einen Träger oder ein Verdünnungsmittel, jeweils physiologisch verträglich, umfaßt.

14. Verwendung von 1,4,5,6-Tetrahydro-2-[α-(o-tolyloxy)benzyl]-pyrimidinhydrochlorid zur Herstellung eines Arzneimittels, das bei der Behandlung von Depressionen nützlich ist.

15. Verwendung von 1,4,5,6-Tetrahydro-2-[α-(o-tolyloxy)benzyl]-pyrimidinhydrochlorid zur Herstellung eines Arzneimittels, das bei der Behandlung von Angst nützlich ist.

16. Verwendung von 1,4,5,6-Tetrahydro-2-[α-(o-tolyloxy)benzyl]-pyrimidinhydrochlorid zur Herstellung eines Arzneimittels, das bei der Behandlung von Bluthochdruck nützlich ist.

## Patentansprüche (Patentansprüche für folgende(n) Vertragsstaat(en): GR, ES)

1. Verbindung der Formel: 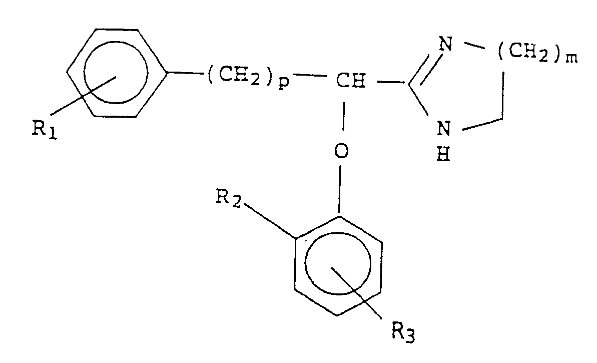 in der R₁ ein Wasserstoff- oder Halogenatom, einen C₁₋₄-Alkyl- oder C₁₋₄-Alkoxyrest, eine OH-, CF₃-, CN-, Methylendioxy- oder Ethylendioxygruppe, einen C₃₋₅-Methylenrest, eine 2,3-Benzo- oder 3,4-Benzogruppe, einen Alkylthio-, Alkylsulfonyl-, Alkylsulfinyl-, C₃₋₆-Cycloalkyl-, C₅₋₈-Cycloalkoxy- oder C₅₋₈-Cycloalkylthiorest darstellt, wobei bis zu 5 monovalente Substituenten am Phenylring auftreten können, die gleich oder verschieden sein können, und falls R₁ einen divalenten Substituenten darstellt, kann einer dieser Substituenten an den Phenylring gebunden sein, der gegebenenfalls mit einem weiteren monovalenten Nichtwasserstoffsubstituenten substituiert sein kann; R₂ ein Halogenatom, einen C₁₋₄-Alkyl- oder C₁₋₄-Alkoxyrest, eine OH-, CF₃- oder CN-Gruppe, einen Alkylthio-, Alkylsulfinyl-, C₃₋₆-Cycloalkyl-, C₅₋₈-Cycloalkoxy- oder C₅₋₈-Cycloalkylthiorest bedeutet; R₃ ein Wasserstoff- oder Halogenatom, einen C₁₋₄-Alkyl- oder C₁₋₄-Alkoxyrest, eine OH-, CF₃-, CN-, Methylendioxy oder Ethylendioxygruppe, einen C₃₋₅-Methylenrest, eine 2,3-Benzo-, 3,4-Benzo-, 4,5-Benzo- oder 5,6-Benzogruppe, einen Alkylthio-, Alkylsulfonyl-, Alkylsulfinyl-, C₃₋₆-Cycloalkyl-, C₅₋₈-Cycloalkoxy- oder C₅₋₈-Cycloalkylthiorest darstellt, R₃ bis zu 4 monovalente Substituenten, die gleich oder verschieden sein können, oder einen einzigen divalenten Substituenten bedeuten kann; m die ganze Zahl 1 oder 2 darstellt; p eine ganze Zahl von 0 - 4 bedeutet; und die pharmazeutisch verträglichen Additionssalze davon,
wobei die Verbindung 1,4,5,6-Tetrahydro-2-[α-(o-tolyloxy)benzyl]-pyrimidinhydrochlorid ausgeschlossen ist.

2. Verbindung nach Anspruch 1, wobei m 1 bedeutet.

3. Verbindung nach Anspruch 1, wobei m 2 darstellt.

4. Verbindung nach einem der Ansprüche 1 bis 3, wobei p 1 bedeutet.

5. Verbindung nach einem der Ansprüche 1 bis 4, wobei R₂ einen Alkoxyrest darstellt.

6. Verbindung nach einem der Ansprüche 1 bis 4, wobei R₂ einen Ethoxyrest bedeutet.

7. Verbindung nach einem der Ansprüche 1 bis 6, wobei R₃ ein Wasserstoffatom darstellt.

8. Verfahren zur Herstellung einer Verbindung der Formel I, wie sie in Anspruch 1 definiert ist, umfassend:
A) Herstellung eines Oxyesters, wie er durch Formel IV beschrieben wird: 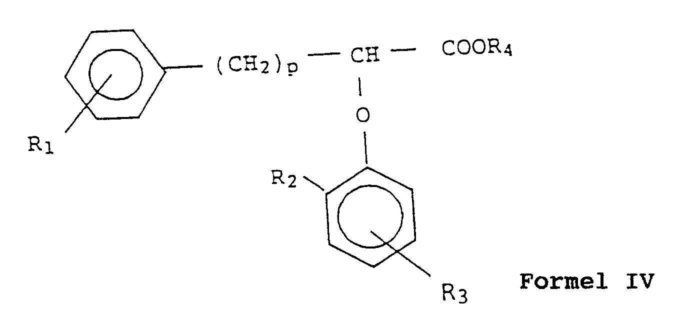 in der R₁, p, R₂, und R₃ wie vorstehend sind und R₄ einen C₁₋₄-Alkylrest darstellt, entweder durch Verfahren #1 oder #2:
1) Ausführung einer Alkylierungsreaktion zwischen einem Bromester, wie er durch Formel II beschrieben wird: 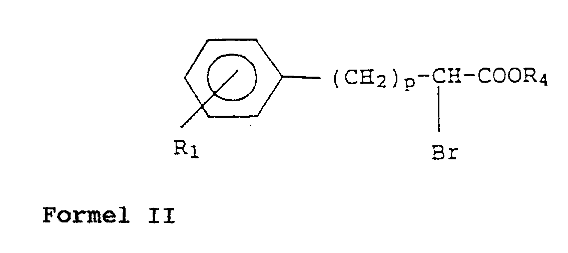 in der p und R₁ wie vorstehend sind und R₄ einen C₁₋₄-Alkylrest darstellt, und einem Phenolderivat, wie es durch Formel III beschrieben wird: 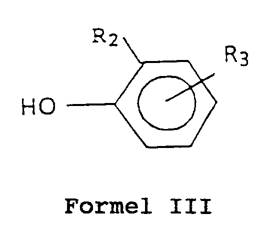 in der R₂ und R₃ wie vorstehend sind,
oder
2)
a) Unterwerfen eines Phenolderivats, wie es durch Formel III beschrieben wird: 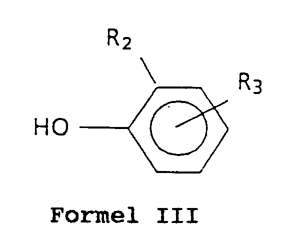 in der R₂ und R₃ wie vorstehend sind, einer Verdrängungsreaktion mit Chlormalonsäurediethylester, wobei das Phenoxyderivat der Formel VI erzeugt wird: 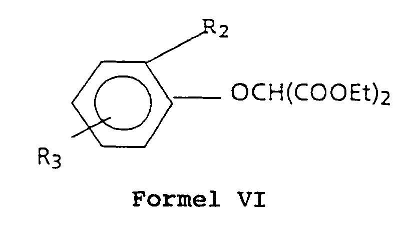 in der R₂ und R₃ wie vorstehend sind,
b) Unterwerfen des erhaltenen Phenoxyderivats einer Verdrängungsreaktion mit einem Chloralkylphenylderivat, wie es durch Formel VII beschrieben wird: 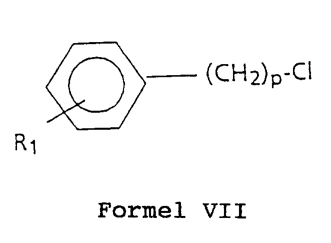 in der R₁ und p wie vorstehend sind, wodurch das Zwischenprodukt der Formel VIII erzeugt wird: 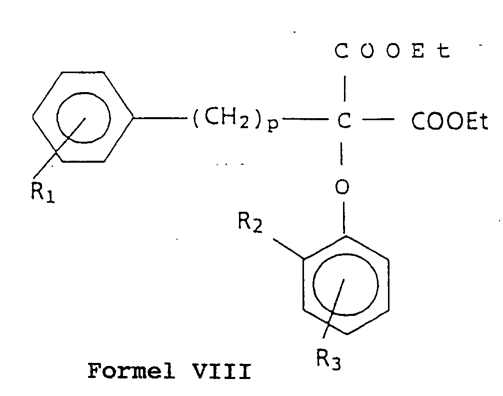 in der R₁, R₂, R₃ und p wie vorstehend sind,
und
c) Unterwerfen dieses Zwischenprodukts einer Decarbethoxylierungsreaktion die den gewünschten Oxyester der Formel IV, in der R₄ eine Ethylgruppe bedeutet, erzeugt,
und
B) Unterwerfen des erhaltenen Oxyesters der Formel IV einer Amidierung und Cyclisierung mit einem Diamin, wie es durch Formel V veranschaulicht wird:
H₂N-CH₂-(CH₂)ₘ-NH₂ Formel V
in der m wie vorstehend ist.

9. Verfahren zur Herstellung eines Arzneimittels, das das Kombinieren einer Verbindung, wie sie in einem der Ansprüche 1 bis 7 definiert ist, mit einem pharmazeutisch verträglichen Träger umfaßt.

10. Verfahren nach Anspruch 9, wobei das hergestellte Arzneimittel zur Behandlung von Depressionen dient.

11. Verfahren nach Anspruch 9, wobei das hergestellte Arzneimittel zur Behandlung von Angst dient.

12. Verfahren nach Anspruch 9, wobei das hergestellte Arzneimittel zur Behandlung von Bluthochdruck dient.

## Revendications (Revendications pour l'(les) Etat(s) contractant(s) suivant(s): AT, BE, CH, DE, DK, FR, GB, IT, LI, LU, NL, SE)

1. Composé de formule : 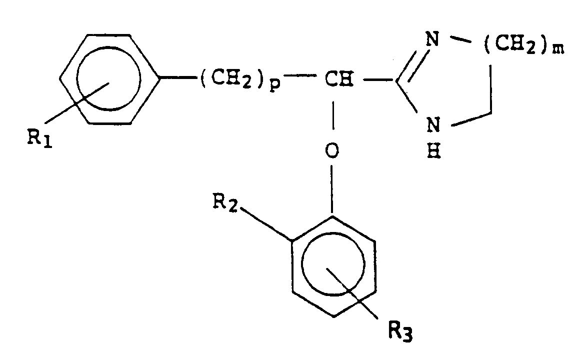 dans laquelle R₁ représente un atome d'hydrogène, un atome d'halogène, un groupement alkyle en C₁-C₄, un groupement alcoxy en C₁-C₄, OH, CF₃, CN, un groupement méthylènedioxy, un groupement éthylènedioxy, un groupement méthylène en C₃-C₅, un groupement 2,3-benzo, un groupement 3,4-benzo, un groupement alkylthio, un groupement alkylsulfonyle, un groupement alkylsulfinyle, un groupement cycloalkyle en C₃-C₆, un groupement cycloalcoxy en C₅-C₈ ou un groupement cycloalkylthio en C₅-C₈ ; il peut y avoir jusqu'à 5 substituants de valence 1 apparaissant sur le noyau phényle qui peuvent être identiques ou différents si R₁ est à représenter par un substituant de valence 2, il peut y avoir l'un de ces substituants lié au noyau phényle qui peut être éventuellement substitué par un autre substituant de valence 1 différent de l'hydrogène ; R₂ représente un atome d'halogène, un groupement alkyle en C₁-C₄, un groupement alcoxy en C₁-C₄, OH, CF₃, CN, un groupement alkylthio, un groupement alkylsulfinyle, un groupement cycloalkyle en C₃-C₆, un groupement cycloalcoxy en C₅-C₈ ou un groupement cycloalkylthio en C₅-C₈ ; R₃ représente un atome d'hydrogène, un atome d'halogène, un groupement alkyle en C₁-C₄, un groupement alcoxy en C₁-C₄, OH, CF₃, CN, un groupement méthylènedioxy, un groupement éthylènedioxy, un groupement méthylène en C₃-C₅, un groupement 2,3-benzo, un groupement 3,4-benzo, un groupement 4,5-benzo, un groupement 5,6-benzo, un groupement alkylthio, un groupement alkylsulfonyle, un groupement alkylsulfinyle, un groupement cycloalkyle en C₃-C₆, un groupement cycloalcoxy en C₅-C₈ ou un groupement cycloalkylthio en C₅-C₈ ; R₃ peut représenter jusqu'à 4 substituants de valence 1 qui peuvent être identiques ou différents ou un substituant unique de valence 2 ; m représente le nombre entier 1 ou 2 ; p représente un nombre entier compris entre 0 et 4 ; et ses sels d'addition pharmaceutiquement acceptables,
à l'exclusion du composé chlorhydrate de 1,4,5,6-tétrahydro-2-[α-(o-tolyloxy)benzyl]pyrimidine.

2. Composé selon la revendication 1, pour lequel m représente 1.

3. Composé selon la revendication 1, pour lequel m représente 2.

4. Composé selon l'une quelconque des revendications 1 à 3, pour lequel p représente 1.

5. Composé selon l'une quelconque des revendications 1 à 4, pour lequel R₂ représente un groupement alcoxy.

6. Composé selon l'une quelconque des revendications 1 à 4, pour lequel R₂ représente un groupement éthoxy.

7. Composé selon l'une quelconque des revendications 1 à 6, pour lequel R₃ représente un atome d'hydrogène.

8. Procédé de préparation d'un composé de formule I tel que défini dans la revendication 1, comprenant les étapes consistant à :
A) produire un ester à groupement oxy tel que décrit par la formule IV : 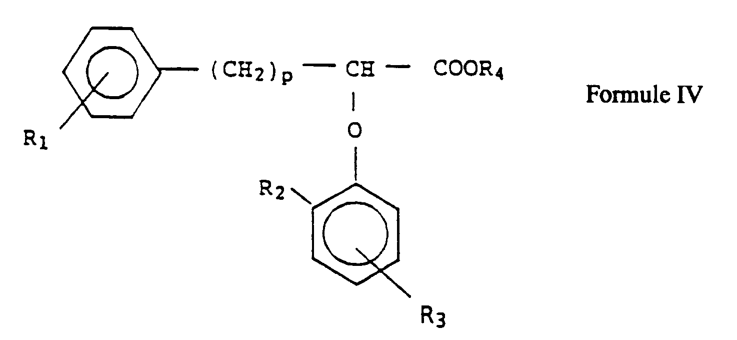 dans laquelle R₁, p, R₂ et R₃ sont tels que ci-dessus et R₄ représente un groupement alkyle en C₁-C₄, soit par le procédé n° 1, soit par le procédé n° 2 :
1) en mettant en oeuvre une réaction d'alkylation entre un ester à groupement bromo tel que décrit par la formule II : 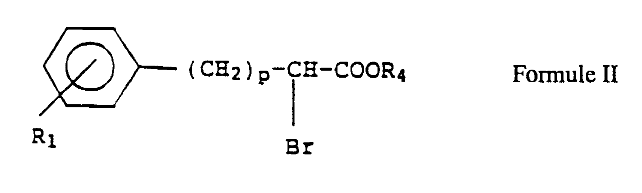 dans laquelle p et R₁ sont tels que ci-dessus et R₄ représente un groupement alkyle en C₁-C₄, et un dérivé du phénol tel que décrit par la formule III : 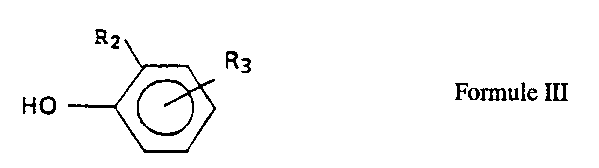 dans laquelle R₂ et R₃ sont tels que ci-dessus,
ou
2)
a) en soumettant un dérivé du phénol tel que décrit par la formule III : 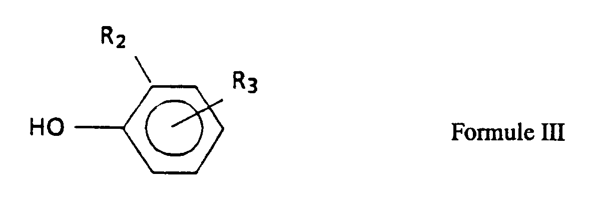 dans laquelle R₂ et R₃ sont tels que ci-dessus, à une réaction de déplacement avec du chloromalonate de diéthyle, obtenant de ce fait le dérivé à groupement phénoxy de formule VI : 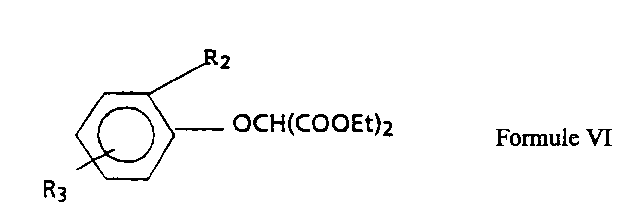 dans laquelle R₂ et R₃ sont tels que ci-dessus,
b) en soumettant le dérivé à groupement phénoxy résultant à une réaction de déplacement avec un dérivé d'un chloroalkylphényle tel que décrit par la formule VII : 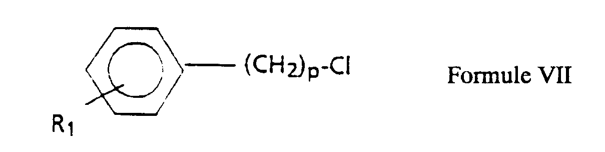 dans laquelle R₁ et p sont tels que ci-dessus, ce qui produit le produit intermédiaire de formule VIII : 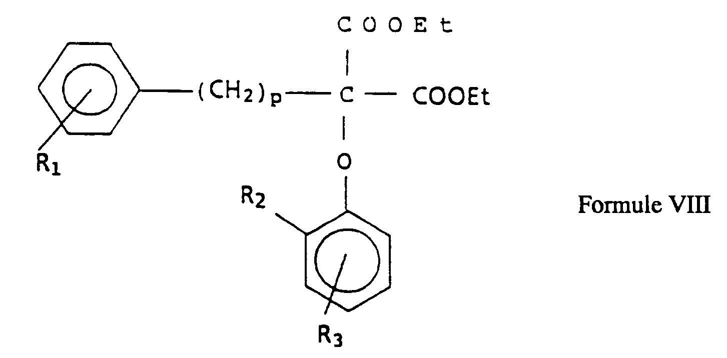 dans laquelle R₁, R₂, R₃ et p sont tels que ci-dessus,
et
c) en soumettant ce produit intermédiaire à une réaction de décarbéthoxylation qui produit l'ester à groupement oxy souhaité de formule IV, dans laquelle R₄ représente le groupement éthyle,
et
B) soumettre l'ester à groupement oxy résultant de formule IV à une amidation et à une cyclisation avec une diamine telle que décrite par la formule V :
H₂N-CH₂-(CH₂)ₘ-NH₂ Formule V
dans laquelle m est tel que ci-dessus.

9. Composé selon l'une quelconque des revendications 1 à 7 à utiliser en tant qu'agent thérapeutiquement actif.

10. Composé selon la revendication 9 pour le traitement de la dépression.

11. Composé selon la revendication 9 pour le traitement de l'anxiété.

12. Composé selon la revendication 9 pour le traitement de l'hypertension.

13. Composition pharmaceutique comprenant un composé selon l'une quelconque des revendications 1 à 7 et un support ou diluant physiologiquement acceptable.

14. Utilisation du chlorhydrate de 1,4,5,6-tétrahydro-2-[α-(tolyloxy)benzyl]pyrimidine pour la préparation d'une composition pharmaceutique utile dans le traitement de la dépression.

15. Utilisation du chlorhydrate de 1,4,5,6-tétrahydro-2-[α-(o-tolyloxy)benzyl]pyrimidine pour la préparation d'une composition pharmaceutique utile dans le traitement de l'anxiété.

16. Utilisation du chlorhydrate de 1,4,5,6-tétrahydro-2-[α-(o-tolyloxy)benzyl]pyrimidine pour la préparation d'une composition pharmaceutique utile dans le traitement de l'hypertension.

## Revendications (Revendications pour l'(les) Etat(s) contractant(s) suivant(s): GR, ES)

1. Composé de formule : 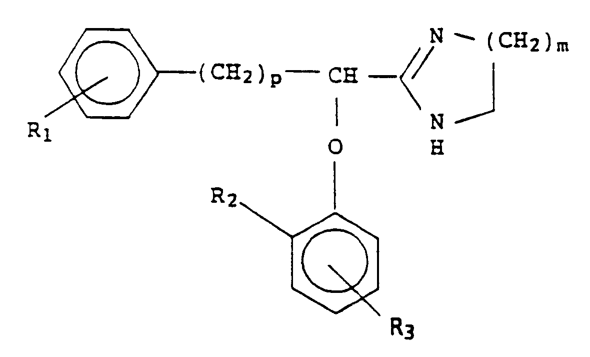 dans laquelle R₁ représente un atome d'hydrogène, un atome d'halogène, un groupement alkyle en C₁-C₄, un groupement alcoxy en C₁-C₄, OH, CF₃, CN, un groupement méthylènedioxy, un groupement éthylènedioxy, un groupement méthylène en C₃-C₅, un groupement 2,3-benzo, un groupement 3,4-benzo, un groupement alkylthio, un groupement alkylsulfonyle, un groupement alkylsulfinyle, un groupement cycloalkyle en C₃-C₆, un groupement cycloalcoxy en C₅-C₈ ou un groupement cycloalkylthio en C₅-C₈ ; il peut y avoir jusqu'à 5 substituants de valence 1 apparaissant sur le noyau phényle qui peuvent être identiques ou différents si R₁ est à représenter par un substituant de valence 2, il peut y avoir l'un de ces substituants lié au noyau phényle qui peut être éventuellement substitué par un autre substituant de valence 1 différent de l'hydrogène ; R₂ représente un atome d'halogène, un groupement alkyle en C₁-C₄, un groupement alcoxy en C₁-C₄, OH, CF₃, CN, un groupement alkylthio, un groupement alkylsulfinyle, un groupement cycloalkyle en C₃-C₆, un groupement cycloalcoxy en C₅-C₈ ou un groupement cycloalkylthio en C₅-C₈ ; R₃ représente un atome d'hydrogène, un atome d'halogène, un groupement alkyle en C₁-C₄, un groupement alcoxy en C₁-C₄, OH, CF₃, CN, un groupement méthylènedioxy, un groupement éthylènedioxy, un groupement méthylène en C₃-C₅, un groupement 2,3-benzo, un groupement 3,4-benzo, un groupement 4,5-benzo, un groupement 5,6-benzo, un groupement alkylthio, un groupement alkylsulfonyle, un groupement alkylsulfinyle, un groupement cycloalkyle en C₃-C₆, un groupement cycloalcoxy en C₅-C₈ ou un groupement cycloalkylthio en C₅-C₈ ; R₃ peut représenter jusqu'à 4 substituants de valence 1 qui peuvent être identiques ou différents ou un substituant unique de valence 2 ; m représente le nombre entier 1 ou 2 ; p représente un nombre entier compris entre 0 et 4 ; et ses sels d'addition pharmaceutiquement acceptables,
à l'exclusion du composé chlorhydrate de 1,4,5,6-tétrahydro-2-[α-(o-tolyloxy)benzyl]pyrimidine.

2. Composé selon la revendication 1, pour lequel m représente 1.

3. Composé selon la revendication 1, pour lequel m représente 2.

4. Composé selon l'une quelconque des revendications 1 à 3, pour lequel p représente 1.

5. Composé selon l'une quelconque des revendications 1 à 4, pour lequel R₂ représente un groupement alcoxy.

6. Composé selon l'une quelconque des revendications 1 à 4, pour lequel R₂ représente un groupement éthoxy.

7. Composé selon l'une quelconque des revendications 1 à 6, pour lequel R₃ représente un atome d'hydrogène.

8. Procédé de préparation d'un composé de formule I tel que défini dans la revendication 1, comprenant les étapes consistant à :
A) produire un ester à groupement oxy tel que décrit par la formule IV : 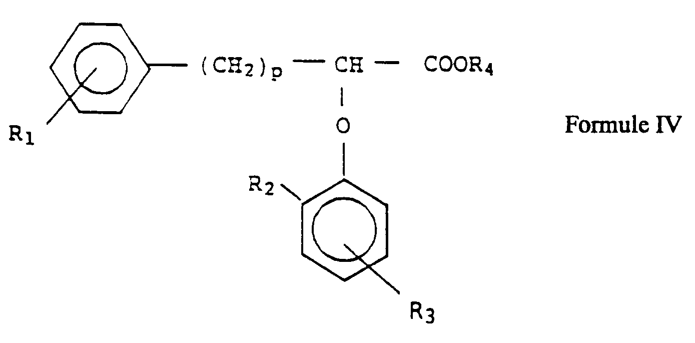 dans laquelle R₁, p, R₂ et R₃ sont tels que ci-dessus et R₄ représente un groupement alkyle en C₁-C₄, soit par le procédé n° 1, soit par le procédé n° 2 :
1) en mettant en oeuvre une réaction d'alkylation entre un ester à groupement bromo tel que décrit par la formule II : 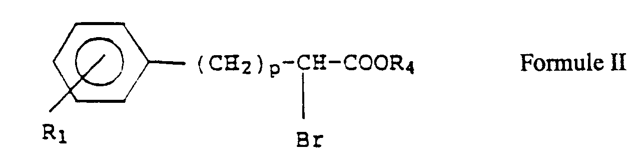 dans laquelle p et R₁ sont tels que ci-dessus et R₄ représente un groupement alkyle en C₁-C₄, et un dérivé du phénol tel que décrit par la formule III : 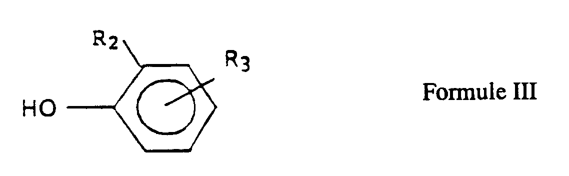 dans laquelle R₂ et R₃ sont tels que ci-dessus,
ou
2)
a) en soumettant un dérivé du phénol tel que décrit par la formule III : 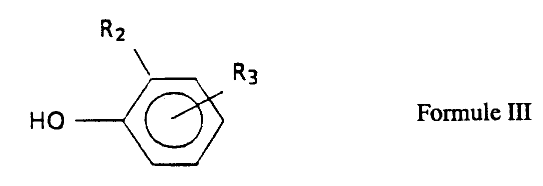 dans laquelle R₂ et R₃ sont tels que ci-dessus, à une réaction de déplacement avec du chloromalonate de diéthyle, obtenant de ce fait le dérivé à groupement phénoxy de formule VI : 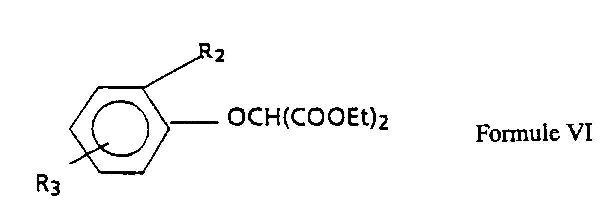 dans laquelle R₂ et R₃ sont tels que ci-dessus,
b) en soumettant le dérivé à groupement phénoxy résultant à une réaction de déplacement avec un dérivé d'un chloroalkylphényle tel que décrit par la formule VII : 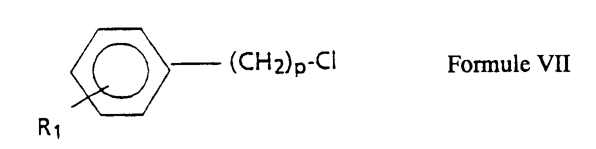 dans laquelle R₁ et p sont tels que ci-dessus, ce qui produit le produit intermédiaire de formule VIII : 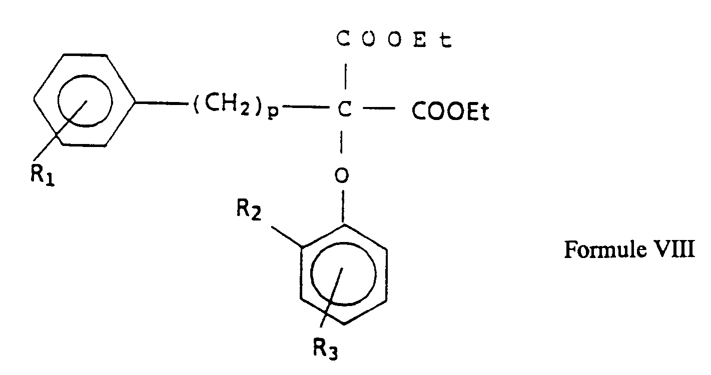 dans laquelle R₁, R₂, R₃ et p sont tels que ci-dessus,
et
c) en soumettant ce produit intermédiaire à une réaction de décarbéthoxylation qui produit l'ester à groupement oxy souhaité de formule IV, dans laquelle R₄ représente le groupement éthyle,
et
B) soumettre l'ester à groupement oxy résultant de formule IV à une amidation et à une cyclisation avec une diamine telle que décrite par la formule V :
H₂N-CH₂-(CH₂)ₘ-NH₂ Formule V
dans laquelle m est tel que ci-dessus.

9. Procédé de préparation d'une composition pharmaceutique comprenant l'association d'un composé tel que défini dans l'une quelconque des revendications 1 à 7 avec un support pharmaceutiquement acceptable.

10. Procédé selon la revendication 9, dans lequel la composition pharmaceutique préparée est destinée au traitement de la dépression.

11. Procédé selon la revendication 9, dans lequel la composition pharmaceutique préparée est destinée au traitement de l'anxiété.

12. Procédé selon la revendication 9, dans lequel la composition pharmaceutique préparée est destinée au traitement de l'hypertension.
